# EUROPEAN PATENT SPECIFICATION

(11) **EP 4 588 452 B1**
(45) Date of publication and mention of the grant of the patent: **04.11.2026**
(21) Application number: 23864300.1
(22) Date of filing: 24.03.2023
(51) Int. Cl.: A61B 34/30, A61B 34/00

(54) **SURGICAL ROBOT SYSTEM AND FLEXIBLE SURGICAL INSTRUMENT**
CHIRURGISCHES ROBOTERSYSTEM UND FLEXIBLES CHIRURGISCHES INSTRUMENT
SYSTÈME DE ROBOT CHIRURGICAL ET INSTRUMENT CHIRURGICAL FLEXIBLE

(30) Priority: 14.09.2022 CN 202211117679
(43) Date of publication of application: 23.07.2025
(73) Proprietor: Beijing Yunlijing'an Technology CO., Ltd, Beijing 102600 (CN)
(72) Inventor: JIANG, Wei, Beijing 102600 (CN); WU, Wenjie, Beijing 102600 (CN); LU, Tianwei, Beijing 102600 (CN)
(74) Representative: Grünecker Patent- und Rechtsanwälte PartG mbB
(86) International application number: PCT/CN2023/083648
(87) International publication number: WO 2024/055557

(56) References cited:
- CN-A- 111 281 544
- CN-A- 111 887 994
- CN-A- 112 043 387
- CN-A- 113 813 050
- CN-A- 114 870 203
- CN-A- 114 870 203
- CN-A- 115 192 200
- CN-A- 115 363 648
- CN-A- 115 363 649
- CN-U- 201 703 910
- CN-U- 216 360 328
- CN-U- 216 360 328
- US-A1- 2004 153 062
- US-A1- 2005 203 382
- US-A1- 2013 303 892
- US-A1- 2017 151 027
- US-A1- 2019 388 164

## Description

### FIELD

The present application relates to the technical field of medical devices, and in particular to a surgical robot system and a flexible surgical instrument.

### BACKGROUND

Diseases of natural orifices such as the digestive system, the urinary system, and the respiratory system are common major chronic diseases. Diseases such as gastric cancer, esophageal cancer, colorectal cancer, bladder cancer, and lung cancer have high incidence and mortality rates, which severely endanger human health. Diagnosis and treatment using flexible endoscopes in cooperation with related surgical instruments have become the mainstream treatment methods, and have the characteristics of small trauma, less bleeding, and low incidence of complications.

It is well known that, unlike conventional large incision surgeries, the operating space for interventional surgeries through body orifices is usually narrow, and thus typically relies on flexible instruments for diagnosis and treatment. There are many existing types of flexible instruments including but not limited to forceps type, electrocoagulation and electrocution type, injection type, and guidance type, which can meet different operational requirements in confined environments. Most of the current surgical instruments are designed based on manual operation. In order to meet the needs of orifice intervention, the existing flexible instruments are designed as flexible and slender instruments, which require the coordination of medical personnel and are complicated to operate in use. In addition, the flexible and slender instruments may easily contact with contaminations, posing a risk of contamination, and there is also the possibility of cross-infection during the instrument retrieval process.

In view of this, the design of the flexible surgical instruments is to be optimized to overcome the above shortcomings.

Relevant prior art is described in US 2019/388164 A1, CN 114 870 203 A and CN 216 360 328 U.

### SUMMARY

An object of the present application is to provide a surgical robot system and a flexible surgical instrument, where a body of an actuator unit of the flexible instrument can be stored through optimized configurations, to meet the operational needs of different application scenarios while achieving the functions of effectively preventing contamination or cross contamination.

In particular, a flexible surgical instrument having the features defined in claim 1 is provided. Moreover, a surgical robot system having the features defined in claim 7 is provided. Further preferred embodiments are defined in the dependent claims. The flexible surgical instrument according to an embodiment of the present application includes a flexible instrument and an instrument drive device that is configured to output a driving force to the flexible instrument. The flexible instrument includes an instrument conveying unit, an actuator unit and a transmission unit. The instrument conveying unit includes a housing and an instrument storage component. The housing has an internal accommodating space, and an instrument outlet is provided on a side wall of the housing. An outer peripheral surface of the instrument storage component is provided with a spiral accommodating groove configured for accommodating a flexible body of the actuator unit in a wound manner. At least part of the instrument storage component is arranged inside the housing and is rotatable and axially movable relative to the housing. The actuator unit includes an actuator and the flexible body. The flexible body includes a drive wire and a sleeve tube in which the drive wire is inserted, and the actuator is arranged at a distal end of the drive wire. The transmission unit includes a transmission base plate that is drivingly connected to the instrument drive device, and the transmission base plate is connected to the instrument storage component of the instrument conveying unit to drive the instrument storage component to rotate and move axially relative to the housing. The actuation transmission assembly includes a traction member and a first drive shaft. The first drive shaft is inserted in the transmission base plate and is drivingly connected to the instrument drive device. The traction member is connected to the first drive shaft, and a proximal end of the drive wire is connected to the traction member. The drive wire is configured to be driven by the traction member to be extended or retracted along a predetermined path, and the drive wire has a rotational degree of freedom relative to the traction member. The rotation transmission assembly includes a rotational shaft, a second terminal, a bevel gear set and a second drive shaft. The second drive shaft is inserted in the transmission base plate and is drivingly connected to the instrument drive device. A driving gear of the bevel gear set is connected to the second drive shaft, and the rotational shaft is connected to a driven gear of the bevel gear set. The drive wire is fixed to the second terminal, and the second terminal is arranged on the rotational shaft. The second terminal is configured to be driven by the rotational shaft to rotate, and the second terminal has a degree of freedom to slide along a traction direction of the drive wire relative to the rotational shaft.

In an embodiment, the transmission unit further includes an actuation transmission assembly and a rotation transmission assembly that are arranged on the transmission base plate. The actuation transmission assembly is configured to be driven by the instrument drive device to extend or retract the drive wire, and the rotation transmission assembly is configured to be driven by the instrument drive device to rotate the drive wire.

In an embodiment, the flexible surgical instrument further includes a docking unit for connecting with an external device. The docking unit includes an electrical connection port, a water connection port and a guide tube. The electrical connection port and the water connection port are arranged at a top of the housing of the instrument conveying unit, and the guide tube is fixed in the housing, to allow a cable and a water pipe to be introduced to the actuator unit through the electrical connection port, the water connection port and the guide tube.

In an embodiment, the instrument drive device includes a first drive component, an output shaft and a drive base plate. The drive base plate mates with the transmission base plate of the transmission unit. The first drive component includes an output end for outputting a rotational driving force. The output shaft is drivingly connected to the output end of the first drive component, and the drive base plate is connected to the output shaft. Two power transmission paths are provided between the output end of the first drive component and the output shaft, and are configured for driving the output shaft to rotate and move axially, respectively, to drive the flexible instrument to perform a conveying operation through the drive base plate.

In an embodiment, a leadscrew nut transmission mechanism and a pulley transmission mechanism are provided between the output end of the first drive component and the output shaft. The power transmission path configured for driving the output shaft to rotate is defined by the pulley transmission mechanism, and the other power transmission path configured for driving the output shaft to move axially is defined by the leadscrew nut transmission mechanism.

In an embodiment, the instrument drive device further includes a second drive component, a third drive component, a first driving transmission disc and a second driving transmission disc. The first driving transmission disc is drivingly connected to an output end of the second drive component, and the second driving transmission disc is drivingly connected to an output end of the second drive component. The drive base plate is provided with a first through hole and a second through hole. The first through hole is arranged corresponding to the first driving transmission disc, and the second through hole is arranged corresponding to the second driving transmission disc, to allow the driving transmission discs drive the actuation transmission assembly and the rotation transmission assembly through the corresponding through holes, respectively.

In an embodiment, the first driving transmission disc and the second driving transmission disc are fixed on two sliding supports, respectively. Each sliding support is axially movable relative to a fixed slide rail provided corresponding to the sliding support, and an elastic reset element is arranged between each sliding support and a fixed structure. The elastic reset element is configured to be deformed when the sliding support moves toward the instrument drive device, to apply a reset force to the sliding support.

In an embodiment, the first driving transmission disc is connected to the second drive component through a first flange, and the second driving transmission disc is connected to the third drive component through a second flange. The first flange and the second flange are fixed on the corresponding sliding supports, respectively.

In an embodiment, an engagement member is provided on the drive base plate, and an engagement groove is provided on the transmission base plate. The engagement member is placed in the engagement groove to form a circumferential rotation limiting pair to transmit the rotational driving force to the instrument side through the engagement member and the engagement groove mating with each other.

In an embodiment, the engagement member includes a hook head portion extending outward from a body of the engagement member, and a side wall of the instrument storage component is provided with an engagement opening mating with the hook head portion. The engagement member is configured to slide relative to the drive base plate to switch between an extended work position and a retracted work position. In a case that the engagement member is at the extended work position, the hook head portion of the engagement member is inserted in the engagement opening. In a case that the engagement member is at the retracted working position, the hook head portion of the engagement member is disengaged from the engagement opening.

In an embodiment, a slide groove is provided on the drive base plate, and the engagement member includes a sliding portion extending downward from a body of the engagement member. The sliding portion mates with the slide groove in a slidable manner, and is configured to slide to switch between the extended work position and the retracted work position. A groove wall of the engagement groove and a groove wall of the slide groove both extend radially.

In an embodiment, the flexible surgical instrument further includes a button and a reset spring. The button is arranged at an outer side end of the engagement member, and the reset spring is pre-compressed and arranged between an inner side end of the engagement member and the slide groove of the drive base plate.

A surgical robot system is further provided according to the present application, including a machine body and the flexible surgical instrument described hereinabove, and the flexible surgical instrument is arranged on the machine body.

In an embodiment, the surgical robot system further includes a human-machine interaction system and an electrical system. The human-machine interaction system includes a touch screen device, a mechanical handle and a display device. The touch screen device and the mechanical handle are arranged at a top of the machine body, and an inclination angle of a display component of the touch screen device is adjustable. The display device is connected to the machine body through a multi-section arm support.

In an embodiment, the touch screen device further includes a wireless handle and a mechanical button that are arranged at the top of the machine body, and the wireless handle includes a manipulation handle and a charging base.

In an embodiment, the machine body includes a housing device, a lifting device and a caster moving device. The housing device includes an upper housing located at a top of the housing device and a lower housing located at a bottom of the housing device, and the lifting device is arranged in the lower housing. The lifting device includes a lifting disc that is movable upward and downward, and the upper housing is arranged on the lifting disc of the lifting device.

In an embodiment, the lifting device includes a lifting drive component, a lifting leadscrew, a motor fixing disc and a load-bearing base disc. The lifting leadscrew is arranged between the lifting disc and the motor fixing disc, and the load-bearing base disc is fixed below the motor fixing disc. A motor is arranged below the load-bearing base disc and is drivingly connected to the lifting leadscrew.

In an embodiment, the housing device further includes an intermediate housing located between the upper housing and the lower housing. The upper housing is inserted in the intermediate housing, and is in a transition fit with the intermediate housing. The upper housing is rotatable around a central axis relative to the intermediate housing. A circumferential limiting pair is provided between a lower portion of the intermediate housing and an upper portion of the lower housing. The lower housing is provided with a heat dissipation hole.

In an embodiment, the lower portion of the intermediate housing is provided with a tooth portion that extends axially, and the upper portion of the lower housing is provided with a groove portion that extends axially. The tooth portion and the groove portion mate with each other to form the circumferential limiting pair.

In an embodiment, the caster moving device includes a caster and a caster lock. The caster lock is arranged on the caster. A bottom plate of the lower housing is provided with an opening corresponding to the caster, and part of a wheel body of the caster is located inside the lower housing.

In an embodiment, the caster is provided with a self-locking electrical feedback interface configured for outputting a caster state signal to a control device of the electrical system. The control device is configured to prevent a starting command from being output to the instrument drive device in a case that the caster state signal indicates that the caster is in an unlocked state.

In an embodiment, the control device of the electrical system is arranged on the bottom plate of the lower housing through a fixing device. The fixing device includes a plurality of fixing claws and a rotatable claw that is located on a side of the fixing device, and the rotatable claw is rotatably connected to the fixing claws through a rotation shaft.

Compared with the conventional technology, an implementation solution, in which the instrument side and the drive side are separate structures assembled with each other, of the flexible surgical instrument is innovatively proposed according to the present application, so as to meet the instrument storage requirements in different application scenarios. The flexible surgical instrument includes the flexible instrument and the instrument drive device that can output the driving force to the flexible instrument. Specifically, the housing of the instrument conveying unit of the flexible instrument has the internal accommodating space and is provided with the instrument outlet on the side wall. The outer peripheral surface of the instrument storage component is provided with the spiral accommodating groove to accommodate the flexible body of the actuator unit in a wound manner, and the instrument storage component can rotate and move axially relative to the housing. Moreover, the transmission unit of the flexible instrument includes the transmission base plate drivingly connected to the instrument drive device, and the transmission base plate is connected to the instrument storage component of the instrument conveying unit to drive the instrument storage component to rotate and move axially relative to the housing. The present solution has the following beneficial technical effects in application.

First, based on the separate configuration of the flexible instrument and the instrument drive device, functional flexible instruments can be provided according to different needs, including but not limited to functional flexible instruments of various sizes, which effectively solves the existing problems of difficult medical coordination and shortage of medical resources and has good adaptability.

Second, along with the rotation of the instrument storage component, the wound flexible body stored in the spiral accommodating groove can be continuously advanced through the instrument outlet. During the advancing process of the actuator, the elastic deformation energy reserved in the deformation caused by the spiral winding can be released, so as to effectively overcome the resistance when entering the flexible endoscope, thereby assisting in providing good advancing capability. When the instrument storage component moves in the opposite direction, the flexible body can be retracted into the housing and spirally wound on the instrument storage component. On the one hand, the flexible body has low rigidity, and can be arranged in an orderly manner due to structural constraints according to this solution, thereby avoiding mutual compression and damage between parts of the body. On the other hand, the retraction and storage of the actuator unit has no space occupancy in the radial direction, and thus the structure is compact and reasonable, which facilitates clinical use and operation.

Third, according to a solution of the present application, the transmission unit further includes the actuation transmission assembly and the rotation transmission assembly that are arranged on the transmission base plate. The actuation transmission assembly is configured to be driven by the instrument drive device to extend or retract the drive wire, and the rotation transmission assembly is configured to be driven by the instrument drive device to rotate the drive wire. That is to say, the transmission unit is provided with an actuation drive interface mating with the instrument drive device, and can achieve operations including but not limited to the traction and rotation of the drive wire.

Fourth, according to another solution of the present application, the flexible instrument has a function of connecting to an external device. Specifically, the docking unit includes the electrical connection port and the water connection port that are arranged at the top of the housing, and further includes the guide tube fixed in the housing, so as to introduce the cable and the water pipe to the actuator unit through the electrical connection port, the water connection port and the guide tube. In this way, the flexible instrument integrates functions of communication with power source, signal source, water and the like, thereby further improving the adaptability in different application scenarios.

Fifth, the surgical robot system according to the present application includes the human-machine interaction system and the electrical system. The human-machine interaction system includes the touch screen device, the mechanical handle and the display device for the output of commands to realize instrument operations, and can display relevant information during surgery including but not limited to the current state of the robot and the information of interaction with the tissue. The touch screen device and the mechanical handle are arranged at the top of the machine body, and the inclination angle of the display component of the touch screen device is adjustable. The actual inclination angle can be adjusted via an internal angle adjustment device, for example, the angle can be adjusted within a range of 10° to 60° but not limited to this. The angle adjustment can cooperate with the lifting and lowering of the entire machine body to adapt to the touch operation angle of the operator.

Sixth, according to still another solution of the present application, the housing device of the machine body includes the upper housing located at the top and the lower housing located at the bottom, and the lifting device is arranged in the lower housing. The lifting device includes the liftable lifting disc, and drives the flexible surgical instrument and the touch screen device on the upper housing to be lifted and lowered through the upper housing arranged on the lifting disc, so as to meet different using needs.

Seventh, according to yet another solution of the present application, the caster is provided with the self-locking electrical feedback interface, and the caster state signal can be output to the control device of the electrical system. The control device prevents the starting command from being output to the instrument drive device in a case that the caster state signal indicates that the caster is in the unlocked state. That is to say, the instrument drive system can only be started when the caster is locked, leading to better safety and reliability.

### BRIEF DESCRIPTION OF THE DRAWINGS

FIG. 1 is a schematic view showing the overall structure of a flexible surgical instrument according to an embodiment of the present application;
FIG. 2 is a schematic view of a flexible instrument according to an embodiment of the present application;
FIG. 3 is an exploded view showing the assembly of the flexible instrument in FIG. 2;
FIG. 4 is a partially sectional schematic view of the flexible instrument in FIG. 2;
FIG. 5 is a schematic view of an actuator unit according to an embodiment of the present application;
FIG. 6 is a sectional view taken along line A-A in FIG. 2;
FIG. 7 is a schematic view showing the overall structure of a transmission unit according to an embodiment of the present application;
FIG. 8 is a schematic view showing the assembly relationship between the transmission unit and an instrument storage component according to an embodiment of the present application;
FIG. 9 is a schematic view showing the assembly relationship between the transmission unit and the instrument storage component from another perspective;
FIG. 10 is a schematic view showing the assembly relationship of an actuation transmission assembly according to an embodiment of the present application;
FIG. 11 is an enlarged view of portion I in FIG. 6;
FIG. 12 is a schematic view of another actuation transmission assembly according to an embodiment of the present application;
FIG. 13 is a schematic view showing the assembly relationship of a rotation transmission assembly according to an embodiment of the present application;
FIG. 14 is an enlarged view of portion II in FIG. 6;
FIG. 15 is a schematic view showing paths of the introduction of power source and signal source according to an embodiment of the present application;
FIG. 16 is a schematic view showing the internal structure of an instrument drive device according to an embodiment of the present application;
FIG. 17 is a schematic view of a connection mechanism at the instrument drive device side in FIG. 16;
FIG. 18 is a schematic view showing the assembly relationship between a drive base plate and the instrument storage component according to an embodiment of the present application;
FIG. 19 is a partial sectional view taken along line B-B in FIG. 17;
FIG. 20 is a schematic view showing the assembly relationship of a first driving transmission disc and a second driving transmission disc according to an embodiment of the present application;
FIG. 21 is a schematic view showing the assembly relationship between a transmission base plate and the transmission unit according to an embodiment of the present application;
FIG. 22 is a schematic view showing the transmission relationship of a first drive component according to an embodiment of the present application;
FIG. 23 is an axial sectional view of FIG. 16;
FIG. 24 is a schematic view showing the connection relationship of a force sensor according to an embodiment of the present application;
FIG. 25 is a schematic view of the flexible instruments according to the embodiment of the present application being used in combination;
FIG. 26 is a schematic view showing the overall structure of a surgical robot system according to an embodiment of the present application;
FIG. 27 is a schematic view showing the structure of a touch screen device according to an embodiment of the present application;
FIG. 28 is a schematic view showing the structure of a mechanical handle according to an embodiment of the present application;
FIG. 29 is a schematic view showing the structure of a wireless handle according to an embodiment of the present application;
FIG. 30 is an exploded view showing the assembly of a machine body according to an embodiment of the present application;
FIG. 31 is a schematic view showing the structure of a lifting device according to an embodiment of the present application;
FIG. 32 is a schematic view showing the assembly relationship among an electrical system, the lifting device and a lower housing according to an embodiment of the present application;
FIG. 33 is a schematic view showing the assembly relationship of a caster moving device according to an embodiment of the present application;
FIG. 34 is a schematic view showing a state where the surgical robot system being lifted according to an embodiment of the present application;
FIG. 35 is a view showing a scenario where a doctor uses a robot while standing;
FIG. 36 is a schematic view showing a state where the surgical robot system is lowered according to an embodiment of the present application; and
FIG. 37 is a view showing a scenario where a doctor uses the robot while seated.

**Reference numerals:**

| | | | |
|---|---|---|---|
| 10 | flexible instrument, | 10a | first flexible instrument, |
| 10b | second flexible instrument, | 11 | instrument conveying unit, |
| 111 | housing, | 1111 | instrument outlet, |
| 1112 | insertion port, | 112 | instrument storage component, |
| 1121 | spiral accommodating groove, | 1122 | through port, |
| 1123 | engagement opening, | 113 | protective tube, |
| 12 | actuator unit, | 12a | first actuator unit, |
| 12b | second actuator unit, | 121 | drive wire, |
| 1211 | limiting block, | 122 | actuator, |
| 123 | sleeve tube, | 13 | transmission unit, |
| 131 | actuation transmission assembly, | 1311 | first drive shaft, |
| 1312 | first terminal, | 13121 | fourth through hole, |
| 1313 | traction member, | 13131 | mounting groove, |
| 1315 | first constraining member, | 13151 | constraining chamber, |
| 13152 | first internal passage, | 1311a | bevel gear set, |
| 1312a | first terminal, | 1313a | leadscrew, |
| 132 | rotation transmission assembly, | 1321 | rotational shaft, |
| 13211 | mounting hole, | 1322 | second terminal, |
| 1323 | bevel gear set, | 1324 | second drive shaft, |
| 1325 | second constraining member, | 13251 | third through hole, |
| 13252 | second internal passage, | 133 | transmission base plate, |
| 1331 | engagement groove, | 134 | first driven transmission disc, |
| 1341 | first recess portion, | 135 | second driven transmission disc, |
| 1351 | second recess portion, | 14 | docking unit, |
| 141 | electrical connection port, | 142 | water connection port, |
| 15 | guide tube, | 161 | signal generator, |
| 162 | signal receiver, | 17 | mounting detection assembly, |
| 20 | instrument drive device, | 21 | first drive component, |
| 22 | second drive component, | 23 | third drive component, |
| 24 | interface component, | 241 | drive base plate, |
| 2411 | first through hole, | 2412 | second through hole, |
| 242 | first driving transmission disc, | 2421 | first protrusion portion, |
| 243 | second driving transmission disc, | 2431 | second protrusion portion, |
| 244 | engagement member, | 2441 | hook head portion, |
| 2442 | guide surface, | 245 | button, |
| 246 | reset spring, | 247 | sleeve, |
| 2471 | covering section, | 2472 | pass-through hole, |
| 248 | connecting sleeve, | 251 | first flange, |
| 252 | second flange, | 253 | sliding support, |
| 254 | slide rail, | 255 | reset element, |
| 261 | leadscrew, | 262 | nut, |
| 263 | driving pulley, | 264 | driven pulley, |
| 265 | output shaft, | 2651 | key, |
| 266 | first shaft sleeve, | 2661 | keyway, |
| 267 | connector, | 268 | bearing fixing seat, |
| 2681 | thrust bearing, | 269 | bearing, |
| 27 | cover housing, | 271 | side wall, |
| 272 | fixing disc, | 273 | second shaft sleeve, |
| 274 | guide sleeve, | 281 | force sensor, |
| 282 | position sensor, | | |
| 100 | flexible surgical instrument, | | |
| 200 | human-machine interaction system, | | |
| 210 | touch screen device, | 2101 | support body, |
| 2102 | display component, | 2103 | touch component, |
| 2104 | base, | 220 | mechanical handle, |
| 2201 | handle joystick, | 2202 | handle button, |
| 230 | wireless handle, | 2301 | manipulation handle, |
| 2302 | charging base, | 240 | mechanical button, |
| 250 | display device, | 2501 | multi-section arm support, |
| 300 | machine body, | 310 | housing device, |
| 3101 | upper housing, | 3102 | intermediate housing, |
| 3103 | lower housing, | 3104 | heat dissipation hole, |
| 3105 | tooth portion, | 3106 | groove portion, |
| 3107 | opening, | 320 | lifting device, |
| 3201 | lifting drive component, | 3202 | lifting leadscrew, |
| 3203 | lifting disc, | 3204 | motor fixing disc, |
| 3205 | load-bearing base disc, | 330 | caster moving device, |
| 3301 | caster, | 3302 | caster lock, |
| 400 | electrical system, | 410 | control device, |
| 420 | fixing device, | 4201 | fixing claw, |
| 4202 | rotatable claw, | 4203 | damping rotation shaft, |
| 430 | cooling fan. | | |

### DETAILED DESCRIPTION OF THE EMBODIMENTS

For those skilled in the art to better understand the technical solutions of the present application, the present application will be further described in detail in conjunction with drawings and embodiments hereinafter.

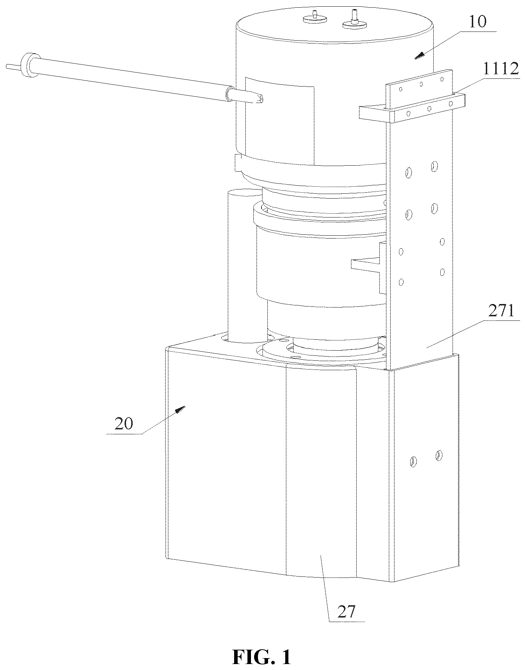

Without loss of generality, a flexible surgical instrument is provided according to the present embodiment, so as to effectively solve the problem that long slender flexible instruments are complicated to operate, difficult to store and easy to be contaminated. Please refer to FIG. 1, which is a schematic view showing the overall structure of the flexible surgical instrument according to the embodiment of the present application.

The flexible surgical instrument 100 includes a flexible instrument 10 and an instrument drive device 20. The flexible instrument 10 is provided with an actuator unit 12 for diagnosis and treatment and for assisting diagnosis and treatment. The instrument drive device 20 provides a driving force to the actuator unit 12 to perform the operations of conveying the flexible instrument and rotating, opening or closing an actuator.

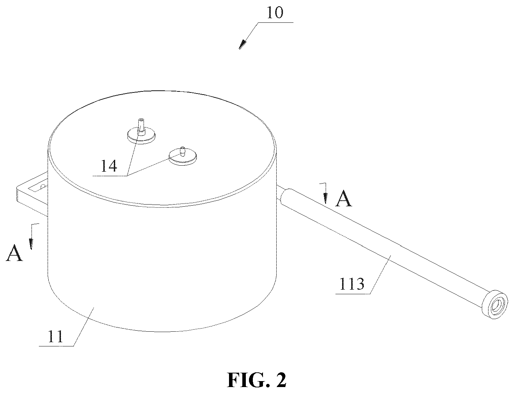

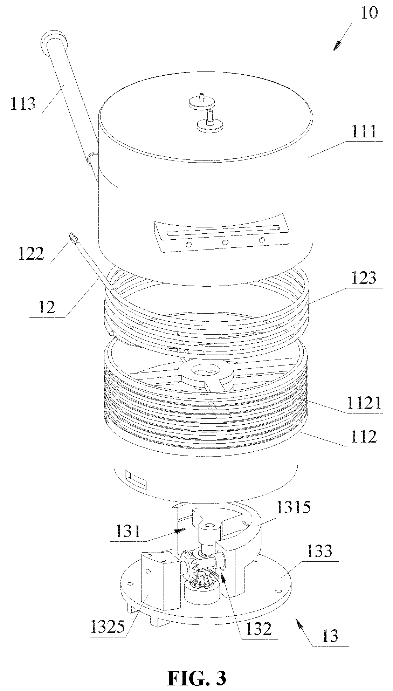

Please refer to FIGS. 2 and 3. FIG. 2 is a schematic view of the flexible instrument according to the embodiment of the present application, and FIG. 3 is an exploded view showing the assembly of the flexible instrument 10 in FIG. 2.

The flexible instrument 10 includes an instrument conveying unit 11, the actuator unit 12 arranged in the instrument conveying unit 11, and a transmission unit 13 for transmitting a driving force for the movement of the actuator.

The instrument conveying unit 11 includes a housing 111 and an instrument storage component 112. Driven by the transmission unit 13, the instrument storage component 112 can rotate relative to the housing 111. After the assembly, the housing 111 is in a relatively fixed state.

A flexible body (a drive wire 121 and a sleeve tube 123) of the actuator unit 12 is wound around a periphery of the instrument storage component 112 and can extend out through an instrument outlet 1111 provided on a side wall of the housing 111. Herein, a protective tube 113 is provided on an outer side of the instrument outlet 1111, and is fixed on the housing 111, so that the actuator unit 12 extending out of the housing 111 is kept in a stable posture. Along with the rotation of the instrument storage component 112, the flexible body of the actuator unit 12 is continuously advanced through the instrument outlet 1111. Similarly, when the instrument storage component 112 moves in an opposite direction, the flexible body can be retracted into the housing and wound around the instrument storage component 112, thereby achieving the retraction and storage of the actuator unit 12. Specifically, in a non-use state, a relatively closed space for storing the body of the flexible instrument is defined by the housing 111 and the instrument storage component 112.

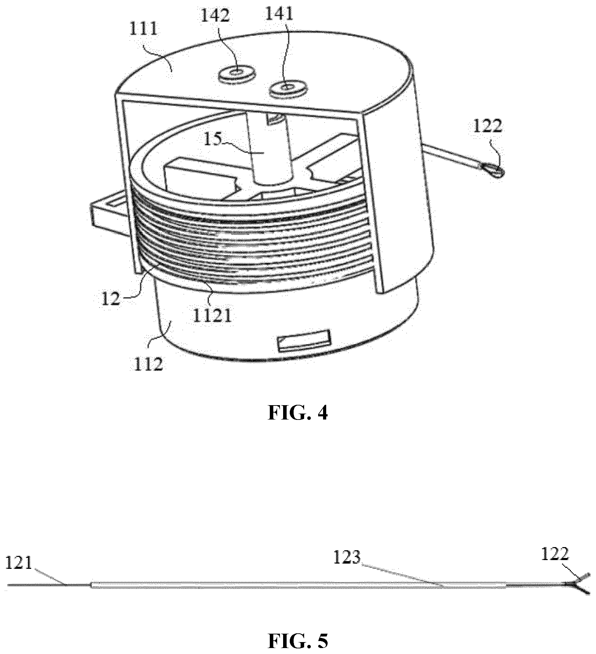

To make the flexible body of the actuator unit 12 be orderly arranged in a wound manner, a spiral accommodating groove 1121 is provided on an outer peripheral surface of the instrument storage component 112. Please refer to FIG. 4, which is a partially sectional schematic view of the flexible instrument in FIG. 2. The flexible body of the actuator unit 12 retracted into the housing is placed in the spiral accommodating groove 1121 of the instrument storage component 112, so as to prevent the flexible body from being disarranged or tangled.

Driven by the transmission unit 13, the instrument storage component 112 can also move axially relative to the housing 111. That is to say, the instrument storage component 112 moves in an axial direction synchronously with its rotation. In this way, the part of the flexible body of the actuator unit 12 detached from the spiral accommodating groove 1121 is substantially aligned with the instrument outlet 1111 in two dimensions, making the retracting and extending operations smooth.

In specific implementations, the actuator unit 12 may be selected according to particular applications, including but not limited to flexible instruments of forceps type, electrocoagulation and electrocution type, basket type, injection type, guidance type, sensor type and the like. The forceps type flexible instrument includes a tissue grasper with a clamping degree of freedom and a hemostat with clamping and rotational degrees of freedom. The electrocoagulation and electrocution type flexible instrument includes a clamping degree of freedom for tissue electrocution and electrocoagulation and a push-pull degree of freedom for a snare instrument. The basket type flexible instrument includes a pushing degree of freedom for pushing out and retracting the basket. The injection type flexible instrument includes a pushing degree of freedom for pushing out and retracting a needle. The guide type flexible instrument is used for coaxial instrument guidance and has no degree of freedom. The sensor type flexible instrument may include an image sensor instrument, a position sensor instrument, or a shape sensor instrument, etc.

The demands of above functions of the actuator unit 12 can be achieved by pulling or rotating a proximal end of the drive wire 121 of the actuator unit 12. Please refer to FIG. 5, which is a schematic view of the actuator unit 12 according to an embodiment of the present application.

Specifically, the actuator 122 at a distal end can be moved by pulling the drive wire 121, allowing operations including but not limited to opening, closing and pushing the actuator. Similarly, the actuator 122 at the distal end can be rotated by turning the drive wire 121.

The directional terms "proximal end" and "distal end" herein are defined from the perspective of the surgical instrument operator. That is, one end of the drive wire 121 close to the operator is the "proximal end", and correspondingly, the other end close to the patient is the "distal end". It should be understood that the above directional terms are only used to describe the technical solution clearly, and do not constitute any substantial limitation on the flexible surgical instrument for which protection is sought in this application.

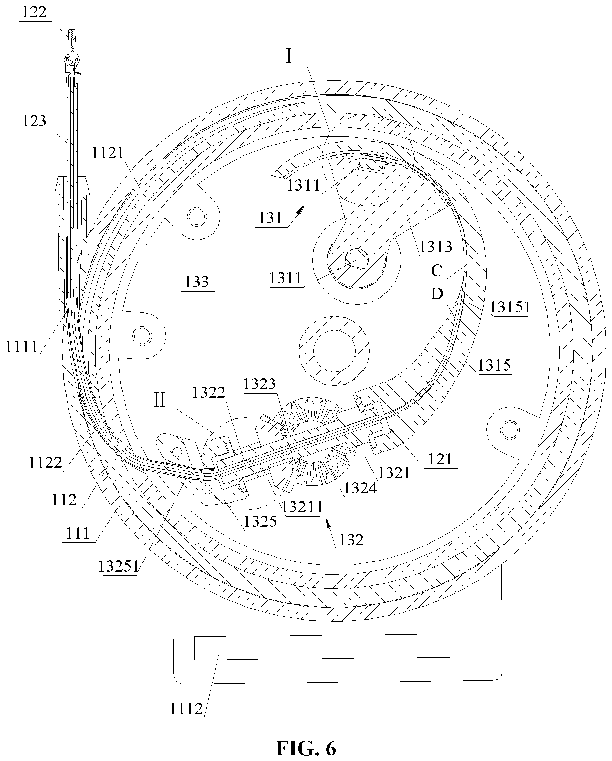

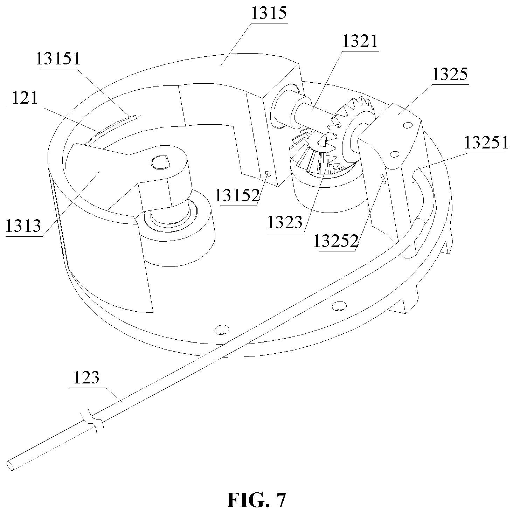

The pulling and rotating of the drive wire 121 of the actuator unit 12 are realized based on the driving force outputted by the instrument drive device 20, and the driving force is specifically transmitted through an actuation transmission assembly 131 and a rotation transmission assembly 132 of the transmission unit 13. Please refer to FIGS. 2, 3, 6 and 7. FIG. 6 is a sectional view taken along line A-A in FIG. 2, and FIG. 7 is a schematic view showing the overall structure of the transmission unit according to an embodiment of the present application.

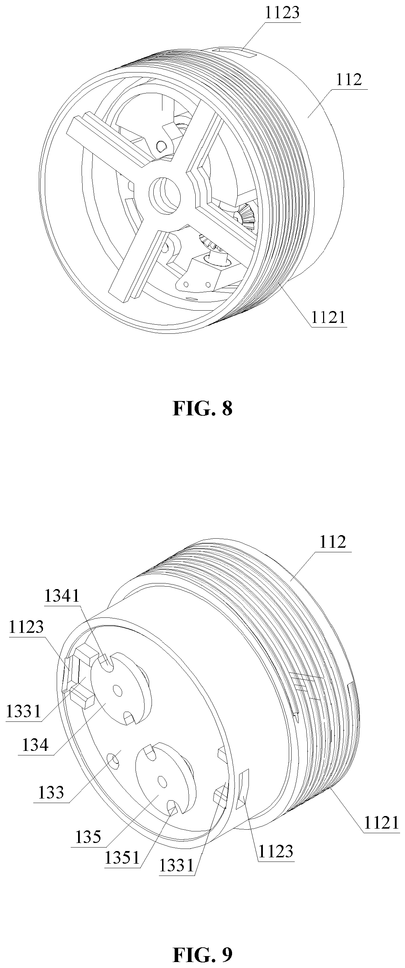

As shown in FIG. 3, the transmission unit 13 includes a transmission base plate 133. The actuation transmission assembly 131 and the rotation transmission assembly 132 are arranged on the transmission base plate 133, and are assembled and fixed to the instrument storage component 112 of the instrument conveying unit 11 through the transmission base plate 133. Please refer to FIGS. 8 and 9, which show the assembly relationship between the transmission unit and the instrument storage component from different perspectives respectively. The overall structure is compact, and is good in the assembly process.

As shown in FIG. 6, the actuation transmission assembly 131 includes a first terminal 1312, a traction member 1313 and a first drive shaft 1311.

The traction member 1313 is connected to the first drive shaft 1311, and the first drive shaft 1311 is inserted in the transmission base plate 133 to rotate under the driving force of the instrument drive device 20 and drive the traction member 1313 to swing around a center of rotation thereof.

The first terminal 1312 is fixed on the traction member 1313 and can move along with the rotation of the traction member 1313. The proximal end of the drive wire 121 is connected to the first terminal 1312. It is configured that the drive wire 121 can be driven by the first terminal 1312 to be extended or retracted along a predetermined path, and the drive wire 121 can rotate relative to the first terminal 1312. It is understandable that a range of swing of the traction member 1313 should satisfy the demand of pulling displacement of the first terminal 1312, that is, the displacement required for extending or retracting the actuator 122 at the distal end.

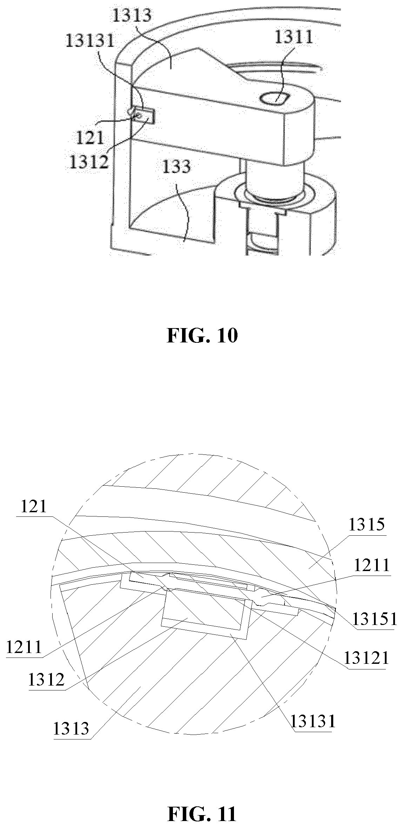

Please refer to FIGS. 10 and 11. FIG. 10 is a schematic view showing the assembly relationship of the actuation transmission assembly according to the embodiment of the present application, and FIG. 11 is an enlarged view of portion I in FIG. 6.

The first terminal 1312 is embedded in the traction member 1313. Specifically, an outer peripheral surface of the traction member 1313 is provided with a mounting groove 13131 mating with the first terminal 1312, and the outer peripheral surface is an arc surface. In other specific implementations, according to actual product design requirements, the shape of the outer peripheral surface of the traction member 1313 is not limited to the arc shape shown in the drawings. Moreover, the fixed terminal may be arranged completely inside the traction member instead of only being embedded on the outer peripheral surface of the traction member 1313 (not shown in the drawings).

In the present embodiment, the drive wire 121 passes through a fourth through hole 13121 of the first terminal 1312, and the body of the drive wire 121 is provided with two limiting blocks 1211, which are respectively located at two ends of the fourth through hole 13121. The size of each of the limiting blocks 1211 is larger than the size of the fourth through hole 13121. When the traction member 1313 rotates forward and reversely, the drive wire 121 can be extended and retracted based on the limiting relationship between the first terminal 1312 and the limiting block 1211 on the corresponding side, thereby meeting the specific operation requirements during the surgery.

Moreover, there is a radial gap between the drive wire 121 and the fourth through hole 13121 of the first terminal 1312, that is, the drive wire 121 has a rotational degree of freedom relative to the fixed terminal. When the drive wire is driven by the rotation transmission assembly 132 to rotate, the drive wire can rotate relative to the fourth through hole 13121 without motion interference with the fixed terminal.

The transmission base plate 133 is a basic component drivingly connected to the drive side, and a first constraining member 1315 is fixedly provided on the transmission base plate 133. The first constraining member 1315 is provided with a constraining chamber 13151, and the drive wire 121 is placed in the constraining chamber 13151. Driven by the first terminal 1312, the drive wire 121 can be extended or retracted along a predetermined path defined by the constraining chamber 13151.

To make full use of the internal space of the housing, the first constraining member 1315 and the constraining chamber 13151 thereon are substantially arc-shaped. Meanwhile, the first constraining member 1315 includes a guide section C and a retaining section D that are connected in sequence. As shown in FIG. 6, the guide section C has an arc-shaped inner wall that mates with the arc-shaped outer peripheral surface of the traction member 1313. A part of the constraining chamber 13151 located on the inner wall of the guide section C is an open channel, while another part of the constraining chamber 13151 at the retaining section D is a closed channel. As such, on the one hand, the displacement of the traction member 1313 can be guided through the arc-shaped inner wall, and on the other hand, the open channel at the guide section C and the closed channel at the retaining section D together form the predetermined path for guiding the drive wire 121.

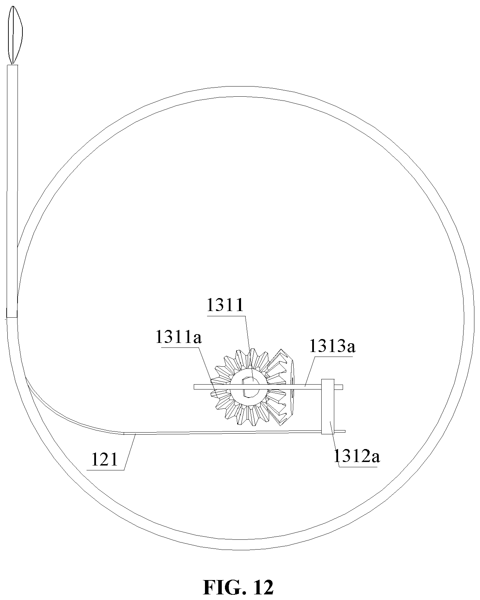

Alternatively, in other specific implementations, the drive wire 12 may be extended or retracted by a first terminal 1312a, a mutually meshing bevel gear set 1311a and a leadscrew 1313a. Please refer to FIG. 12, which is a schematic view of another actuation transmission assembly according to an embodiment of the present application. To clearly illustrate the difference and connection between the present embodiment and the embodiment described in FIG. 6, the same functional components or structures are indicated by the same reference numeral.

As shown in FIG. 12, a driving gear of the bevel gear set 1311a can be driven by the first drive shaft 1311 to drive a driven gear to rotate, and the leadscrew 1313a rotates coaxially with the driven gear. Meanwhile, one end of the first terminal 1312a fixedly clamps the proximal end of the drive wire 121, and the other end of the first terminal 1312a is provided with a nut (not shown in the drawings) in fit with the leadscrew 1313a. Herein, the nut is fixed on the first terminal 1312a and can move axially along with the rotation of the leadscrew 1313a, so as to drive the drive wire 121 to be extended or retracted through the first terminal 1312a. It should be understood that, a constraining chamber for forming a predetermined path may also be provided in the embodiment illustrated in FIG. 12.

As shown in FIGS. 3 and 6, in the present embodiment, the rotation transmission assembly 132 includes a rotational shaft 1321, a second terminal 1322, a bevel gear set 1323 and a second drive shaft 1324.

A driving gear of the bevel gear set 1323 is connected to the second drive shaft 1324, and the second drive shaft 1324 is inserted in the transmission base plate 133 to rotate under the driving force of the instrument drive device 20 and drive the rotational shaft 1321 to rotate around a center of rotation thereof through the bevel gear set 1323.

The second terminal 1322 is arranged on the rotational shaft 1321, and the drive wire 121 is fixed to the second terminal 1322 and can move along with the rotation of the rotational shaft 1321. The second terminal 1322 is configured to be driven by the rotational shaft 1321 to rotate, and the second terminal 1322 is configured to slide relative to the rotational shaft 1321 in a pulling direction of the drive wire 121. Herein, the sliding displacement of the second terminal 1322 also needs to meet the demand of the pulling displacement of the first terminal 1312.

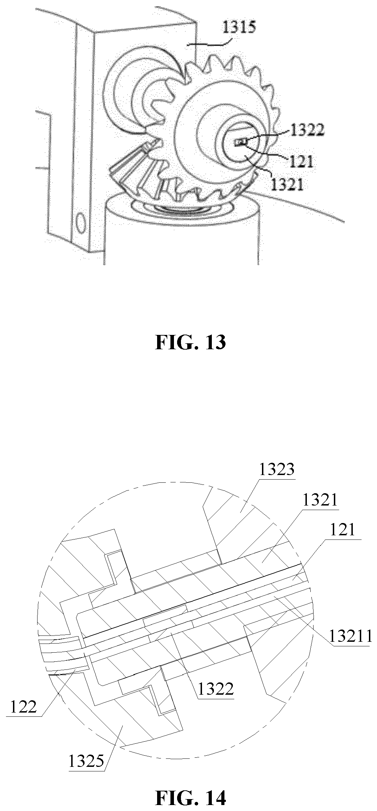

Please refer to FIGS. 13 and 14. FIG. 13 is a schematic view showing the assembly relationship of the rotation transmission assembly according to the embodiment of the present application, and is formed by cutting at the position of the second terminal 1322 along the radial direction. FIG. 14 is an enlarged view of portion II in FIG. 6.

The second terminal 1322 is embedded in the rotational shaft 1321. Specifically, a mounting hole 13211 is provided at a center of the rotational shaft 1321. The drive wire 121 extends to the constraining chamber of the first constraining member through the mounting hole 13211. In the present embodiment, the second terminal 1322 fixedly connected to the drive wire 121 is inserted in the mounting hole 13211, and the second terminal 1322 and the mounting hole 13211 both have rectangular cross-sections matching each other. In this way, when the rotational shaft 1321 rotates, the second terminal 1322 rotates synchronously to drive the drive wire 121 to rotate. Meanwhile, the second terminal 1322 has a sliding degree of freedom relative to the rotational shaft 1321, that is, the second terminal 1322 can move axially relative to the rotational shaft 1321. When being pulled by the actuation transmission assembly 131, the drive wire can rotate relative to the mounting hole 13211 without motion interference with the rotational shaft.

In other specific implementations, the cross-sections of the second terminal 1322 and the mounting hole 13211 may have other structures including but not limited to other polygonal shapes or shapes with circumferential limiting planes. Any solution, which satisfies the functional requirements that the second terminal can slide in the mounting hole and rotate synchronously with the rotational shaft, falls within the scope of protection of the present application.

A second constraining member 1325 is fixedly provided on the transmission base plate 133. As shown in FIGS. 6 and 7, in the axial direction of the rotational shaft 1321, an end of the second constraining member 1325 faces an end of the retaining section D of the first constraining member 1315. The second constraining member 1325 and the first constraining member 1315 provide support to respective shaft ends, so that the shaft ends on both sides of the rotational shaft 1321 are each in a reliable pivotal mating relationship to meet the functional requirements of relative rotation.

Herein, a third through hole 13251 is provided in the second constraining member 1325, and correspondingly, a through port 1122 is provided in the instrument storage component 112 of the instrument conveying unit 11. The through port 1122 is obliquely provided so that the flexible body of the actuator unit 12 extends and transitions to the spiral accommodating groove 1121 on the outer surface of the instrument storage component 112. The diameter of the third through hole 13251 matches the size of the sleeve tube 123 of the flexible body of the actuator unit 12, so as to reliably fix a tube end of the sleeve tube 123.

In addition, the flexible instrument 10 according to the present embodiment further includes a docking unit 14 configured for connections with external devices including but not limited to the connections of power source, signal source and water. Please refer to FIGS. 2 and 4, an electrical connection port 141 and a water connection port 142, both in communication with the interior of the housing 111, are provided at a top end of the housing 111, and a guide tube 15 extending in the axial direction is fixedly provided on the housing 111. The power source and signal source can be introduced into the interior of the instrument through the electrical connection port 141, and the external water source can be introduced into the interior of the instrument through the water connection port 142. The sources enter the actuator unit 12 through the guide tube 15 at the center of the housing 111, and are then connected to the actuator at the distal end.

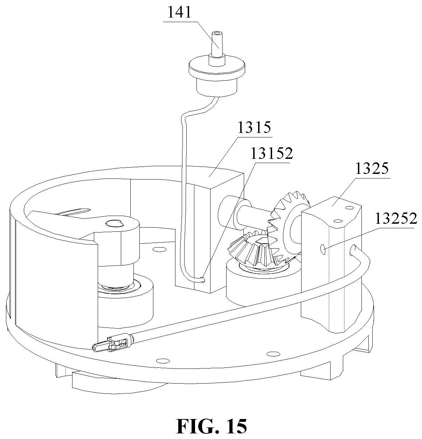

Please refer to FIGS. 7 and 15 illustrating the introduction of the power source and the signal source. FIG. 15 is a schematic view showing paths of the introduction of the power source and the signal source.

The first constraining member 1315 is provided with a first internal passage 13152 in communication with the mounting hole 13211 of the rotational shaft. A cable introduced via the electrical connection port 141 runs downward through the guide tube, enters the mounting hole 13211 of the rotational shaft through the first internal passage 13152 of the first constraining member 1315, and is connected to the actuator at the distal end through the sleeve tube 123 of the actuator unit 12, so as to achieve the functions such as, but not limited to, the power supply of the actuator at the distal end and the signal interaction and transmission with the actuator at the distal end.

The second constraining member 1325 is further provided with a second internal passage 13252 in communication with the third through hole 13251. A water pipe introduced via the water connection port 142 also runs downward through the guide tube, enters the third through hole 13251 through the second internal passage 13252 of the second constraining member 1325, and is connected to the actuator at the distal end through the sleeve tube 123 of the actuator unit 12, so as to achieve the functions such as, but not limited to, the perfusion of irrigation fluids.

In order to facilitate the quick assembly of the entire device, according to the present embodiment, a detachable connection mechanism is provided between the flexible instrument 10 and the instrument drive device 20, which specifically includes detachable connections among the instrument storage component 112, the transmission unit 13 (the actuation transmission assembly 131 and the rotation transmission assembly 132) and the instrument drive device 20, thereby satisfying the demands for achieving functions of transmitting the corresponding driving force while allowing the quick assembly operation.

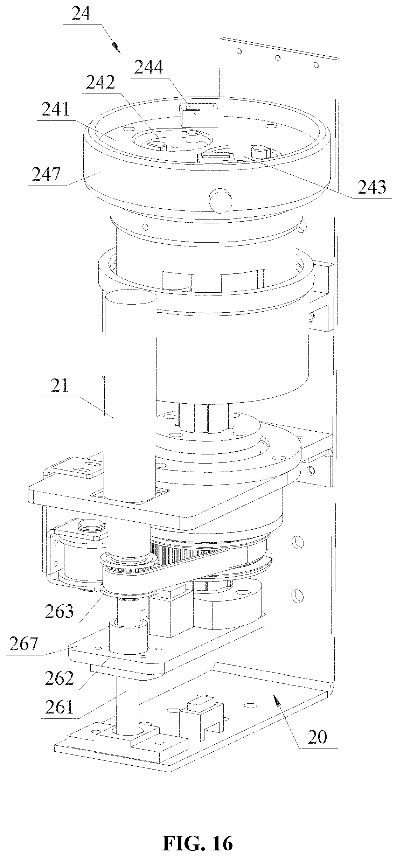

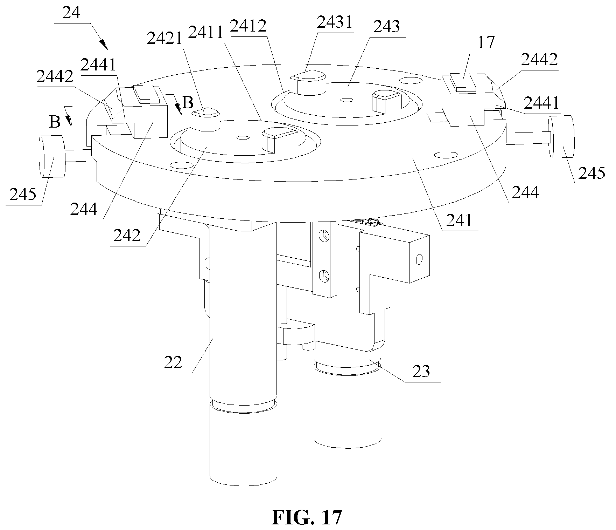

Please refer to FIGS. 16 and 17. FIG. 16 is a schematic view showing the internal structure of the instrument drive device 20 according to an embodiment of the present application, and FIG. 17 is a schematic view of the connection mechanism at the instrument drive device side in FIG. 16.

As shown in the drawings, an interface component 24 for outputting power is provided at a top of the instrument drive device 20. A drive base plate 241 serves as an interface connector for outputting a driving force of a first drive component 21, and is used to transmit power to the instrument storage component 112. A first driving transmission disc 242 serves as an interface connector for outputting the driving force of a second drive component 22 to transmit power to the first drive shaft 1311 to pull the drive wire. A second driving transmission disc 243 serves as an interface connector for outputting the driving force of a third drive component 23 to transmit power to the second drive shaft 1324 to rotate the drive wire.

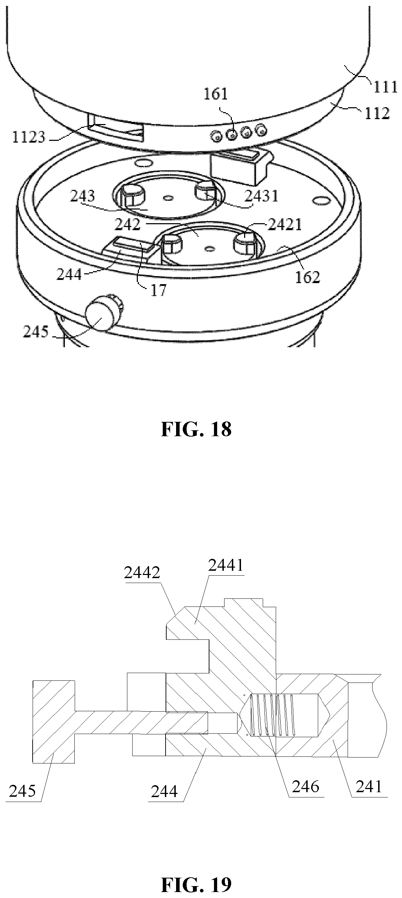

Please refer to FIGS. 8, 9 and 18. FIG. 18 is a schematic view showing the assembly relationship between the drive base plate and the instrument storage component 112.

The drive base plate 241 and the transmission base plate 133 are arranged to face each other. The drive base plate 241 is provided with an engagement member 244, and the transmission base plate 133 is correspondingly provided with an engagement groove 1331. After assembly, the engagement member 244 is arranged in the engagement groove 1331 to form a circumferential rotation limiting pair. When the drive base plate 241 is driven by the first drive component 21 to rotate, the transmission base plate 133 can be driven to rotate synchronously via the circumferential rotation limiting pair, so as to further drive the instrument storage component 112 fixed to the transmission base plate 133 to rotate, to advance the actuator unit 12.

In the present embodiment, two sets of mating engagement members 244 and engagement grooves 1331 are provided, and are symmetrically arranged so that the force is relatively balanced. It is understandable that, in other specific implementations, the sets of mating engagement members 244 and engagement grooves 1331 may be in other plural quantities and are circumferentially spaced apart from each other.

Further, the engagement member 244 can move radially relative to the drive base plate 241, that is, the engagement member 244 can also slide in the engagement groove 1331. The engagement member 244 has a hook head portion 2441 extending outward from the body of the engagement member 244. Accordingly, an engagement opening 1123 adapted to the hook head portion 2441 is provided on a side wall of the instrument storage component 112. As such, when the engagement member 244 is at an extended work position, the hook head portion 2441 is inserted in the engagement opening 1123 to prevent the instrument storage component 112 from being detached.

In this way, when the instrument storage component 112 is driven to rotate, the instrument storage component 112 can be driven through the engagement member 244 and the engagement groove 1331 mating with each other to extend or retract synchronously in the axial direction. In other specific implementations, the demand for achieving functions of the synchronous axial movement may be realized through other structural forms.

In order to improve the operability, a button 245 is provided on the outer side of each engagement member 244, and a reset spring 246 is provided on the inner side of each engagement member 244. The reset spring 246 is arranged between the engagement member 244 and the drive base plate 241 in a pre-compressed state, so that the engagement member 244 can be reliably retained at the extended work position. Please refer to FIG. 19, which is a partial sectional view taken along line B-B in FIG. 17.

During disassembly, the operator applies a force onto the button 245. The engagement member 244 slides inward along the engagement groove 1331, and the reset spring 246 is further deformed. The hook head portion 2441 is disengaged from the engagement opening 1123, so that the flexible instrument 10 can be detached.

A guide surface 2442 is provided at a top of an end of the hook head portion 2441 extending outward, and the guide surface 2442 extends downward. During actual assembly, a lower edge of the instrument storage component 112 axially abuts against the guide surface 2442 of the hook head portion 2441, generating an inward component force applied on the engagement member 244 in the radial direction, and the engagement member 244 slides inward under this force. At the same time, the reset spring 246 is compressed and further deformed. As the instrument storage component 112 moves axially, when the engagement opening 1123 on the instrument storage component 112 is aligned with the hook head portion 2441, the reset spring 246 releases elastic deformation energy, pushing the hook head portion 2441 into the engagement opening 1123, thereby finishing the quick assembly operation of the two parts.

It is understandable that, in other specific implementations, the reset spring may be implemented in other structural forms including but not limited to a reset element made based on the properties of rubber materials, or a reset element having a spring sheet structure.

Further, in order to avoid the possible impact caused by the exposed engagement structure, in the present embodiment, a sleeve 247 is provided at the periphery of the drive base plate 241, and the sleeve 247 includes a covering section 2471 extending upward in the axial direction. The drive base plate 241 and the engagement members 244 thereon are arranged in a cavity defined by the covering section 2471. Moreover, two pass-through holes 2472 are provided on the covering section 2471, which are aligned with the two buttons 245 in the radial direction, respectively, so that push rods of the buttons 245 are fixed to the bodies of the respective engagement members 244 through the respective pass-through holes 2472. In this way, the engagement structure is effectively covered, and the operability of the button can also be ensured.

Herein, the drive base plate 241 is drivingly connected to the first drive component 21 through the sleeve 247 and a connecting sleeve 248. Alternatively, in other implementations, the drive base plate 241 may be drivingly connected to the first drive component 21 directly, or may be drivingly connected to the first drive component 21 through the sleeve 247.

In addition, an electronic identification assembly may be provided between the flexible instrument 10 and the instrument drive device 20 to identify the type of the instrument currently being connected. As shown in FIG. 17, the electronic identification assembly includes a signal generator 161 at the flexible instrument side and a signal receiver 162 at the instrument drive device side. Specifically, the signal generator 161 may be arranged on the outer peripheral surface of the instrument storage component 112, and the signal receiver 162 may be arranged on an inner wall of the sleeve 247 (not shown in the drawings). The identification is achieved based on radio frequency signal identification.

In addition, to monitor the mounting status in real time, a mounting detection assembly 17 is provided at the instrument drive device side. As shown in FIGS. 16 and 17, the mounting detection assembly 17 is a micro switch, and is arranged on a top surface of the engagement member 244. When the flexible instrument is mounted on the drive device, the flexible instrument compresses the micro switch to generate a signal, thereby realizing the mounting status detection.

It should be noted that, the electronic identification assembly and the mounting detection assembly may be in the form of other devices, which may be determined according to actual product design requirements, and are not limited to the device types and arrangement positions shown in the drawings.

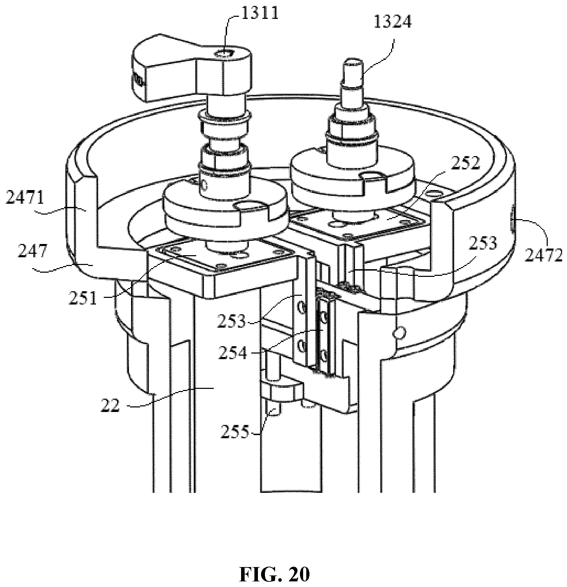

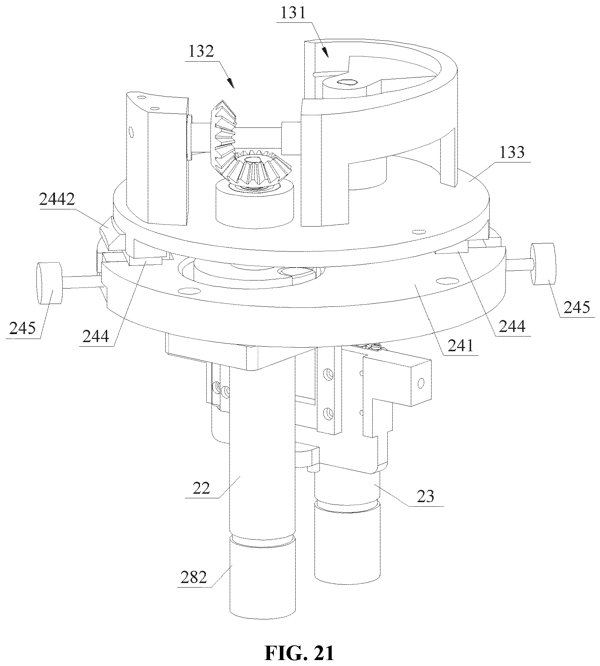

Please refer to FIGS. 9, 17, 18, 20 and 21. FIG. 20 is a schematic view showing the assembly relationship of the first driving transmission disc 242 and the second driving transmission disc 243, and FIG. 21 is a schematic view showing the assembly relationship between the transmission base plate and the transmission unit.

In the present embodiment, two sets of drivingly-connected driving transmission discs and driven transmission discs are provided. A first driven transmission disc 134 and a second driven transmission disc 135 are provided at a bottom of the transmission base plate 133. The first driven transmission disc 134 is fixedly connected to a shaft end of the first drive shaft 1311 and is connected to the first driving transmission disc 242 in a mated manner. The second driven transmission disc 135 is fixedly connected to a shaft end of the second drive shaft 1324 and is connected to the second driving transmission disc 243 in a mated manner.

Accordingly, the drive base plate 241 is provided with a first through hole 2411 and a second through hole 2412, so that the first driving transmission disc 242 and the second driving transmission disc 243 are mated with the corresponding driven transmission discs through the two through holes respectively. Moreover, the second drive component 22 is fixed to a first flange 251, and an output shaft of the second drive component 22 is connected to the first driving transmission disc 242 through the first flange 251. The third drive component 23 is fixed to a second flange 252, and an output shaft of the third drive component 23 is connected to the second driving transmission disc 243 through the second flange 252. The components are basically arranged in sequence along the axial direction, thereby reducing the space occupancy in the radial dimension.

To further match the axial displacements for connection on different connecting sides, the detachable connection mechanism according to the present embodiment further has adaptability in the axial direction. The first flange 251 and the second flange 252 are fixed on corresponding sliding supports 253, respectively, and the two sliding supports 253 can axially move relative to fixed slide rails 254, respectively. Herein, to simplify the illustration, only the sliding support 253 and the slide rail 254 corresponding to the first flange 251 are shown in FIG. 20. That is to say, the sliding support 253 has the degree of freedom to axially move relative to the slide rail 254 to adaptively adjust the relative position in the axial direction.

Accordingly, an elastic reset element 255 is provided at a bottom of each sliding support 253 to apply a reset force to the sliding support 253, so as to establish a reliable connection between the corresponding driving and driven transmission discs. It is understandable that, the slide rail 254 is a relatively fixed structure, and the fixed connection manner may be determined according to the internal space, which may include but not limited to a fixed connection on the connecting sleeve 248 as shown in the drawings.

As shown in FIG. 9, the first driven transmission disc 134 is provided with a first recess portion 1341, and the second driven transmission disc 135 is provided with a second recess portion 1351. As shown in FIGS. 17 and 18, the first driving transmission disc 242 is provided with a first protrusion portion 2421, and the second driving transmission disc 243 is provided with a second protrusion portion 2431, the protrusion portions mate with the recess portions on the driven transmission discs respectively to form circumferential limiting pairs. In this way, when the second drive component 22 and the third drive component 23 are separately started, the power can be transmitted to the drive shafts of the transmission unit through the above mated driving and driven transmission discs respectively to realize the operations of pulling and rotating the actuator unit.

In the present embodiment, outer diameters of the mutually mated driving transmission disc and driven transmission disc are substantially the same. The recess portion is formed by radially recessing inward from the outer peripheral surface of the driven transmission disc, and the protrusion portion is formed by axially protruding from the top surface of the driving transmission disc. After assembly, the protrusion portion on the driving transmission disc is engaged with the recess portion on the driven transmission disc, leading to the characteristics of compact structure and high connection reliability. In other specific implementations, the number of the mutually mated protrusion and recess portions on each transmission path may be determined according to the overall product design requirements, and is not limited to the two sets shown in the drawings.

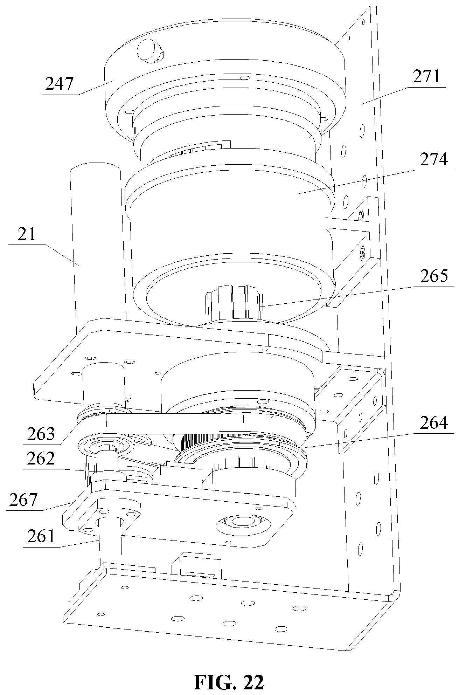

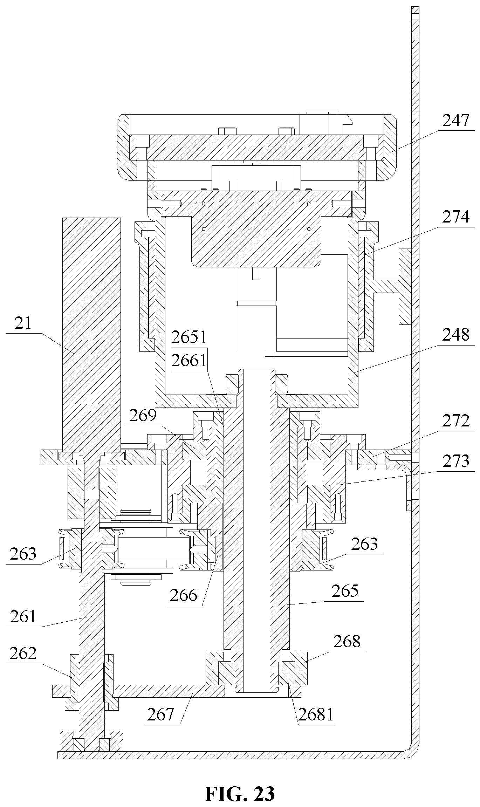

In addition, the rotation and axial displacements of the instrument storage component 112 according to the present embodiment are driven by the first drive component 21 and are respectively achieved through the two power transmission paths. Please refer to FIGS. 16, 22 and 23. FIG. 22 is a schematic view showing the transmission relationship of the first drive component. FIG. 23 is an axial sectional view of FIG. 16 obtained by specifically sectioning along a central line of the first drive component and an output shaft thereof.

The output shaft of the first drive component 21 is coaxially fixed with the leadscrew 261, and the leadscrew 261 serves as the basic transmission component of the two power transmission paths. As shown in the drawings, the leadscrew 261 is provided with a driving pulley 263 and a nut 262 that are spaced apart from each other.

The driving pulley 263 is fixed on the leadscrew 261, and the rotational driving force is transmitted to a driven pulley 264 through a belt. The driven pulley 264 is arranged on an output shaft 265. Specifically, the driven pulley 264 is fixed on a first shaft sleeve 266, and the first shaft sleeve 266 is pivotally connected to a fixed structure through a bearing 269. An outer surface of the output shaft 265 is provided with a key 2651 that is axially arranged, and an inner surface of the first shaft sleeve 266 is provided with a keyway 2661 that mates with the key. With the pulley transmission mechanism, the output shaft 265 is driven by the first shaft sleeve 266 to rotate synchronously, and the output shaft 265 can move axially relative to the first shaft sleeve 266.

The nut 262 is in threaded fit with the leadscrew 261, and is connected to the output shaft 265 through a connector 267. The nut 262 is fixed at one end of the connector 267, and the output shaft 265 is pivotally connected to the other end of the connector 267. The output shaft 265 and the connector 267 are axially constrained relative to each other. Specifically, a thrust bearing 2681 is arranged at a shaft end of the output shaft 265, and a bearing fixing seat 268 is fixed on the connector 267. The bearing fixing seat 268 and the thrust bearing 2681 are axially constrained relative to each other. Based on the fitting relationship between the leadscrew and the nut, the output shaft 265 can be driven to move back and forth in the axial direction.

As such, the driving force output by the output shaft of the first drive component 21 drives the output shaft 265 to rotate and move axially through the two transmission paths synchronously, and drives the drive base plate 241 to rotate and move axially through the connecting sleeve 248 fixed on the output shaft 265. In the above composite motion, the rotational motion is a main motion for conveying the flexible instrument, while the axial motion is an auxiliary motion for ensuring that the flexible body of the actuator unit 12 is aligned with the through port 1122 of the instrument storage component 112.

It should be noted that, the fixed structure to which the first shaft sleeve 266 is pivotally connected and the related structure for keeping the housing 111 fixed can be realized in different ways. In the present embodiment, the above fixed structures are integrated on a cover housing 27 of the instrument drive device 20, and a side wall 271 of the cover housing 27 extends upward to a side of the housing 111 of the flexible instrument 10.

For the fixation of the housing 111 of the flexible instrument 10, please refer to FIGS. 1 and 16. An insertion port 1112 is provided on the outer peripheral surface of the housing 111, and the insertion port 1112 is sleeved on the side wall 271 to mount the housing 111 on the side wall 271. The housing 111 is fixed on the side wall 271 by threaded fasteners according to different assembly sizes.

The first shaft sleeve 266 is pivotally connected to a fixing disc 272. As shown in FIGS. 16 and 23, the fixing disc 272 is fixed to the side wall 271, so that a second shaft sleeve 273 for mounting the bearing 269 is formed and fixed. Alternatively, in other specific implementations, the second shaft sleeve 273 and the fixing disc 272 may form an integrated structure.

Further, in order to improve the stability of the axial movement, a guide sleeve 274 is provided at the periphery of the connecting sleeve 248. The guide sleeve 274 is fixed on the side wall 271, and an axial movement fitting pair is formed by the connecting sleeve 248 and the guide sleeve 274 to provide guide and support within a range of the axial movement of the connecting sleeve 248, thereby ensuring good actuation performance of the related structure.

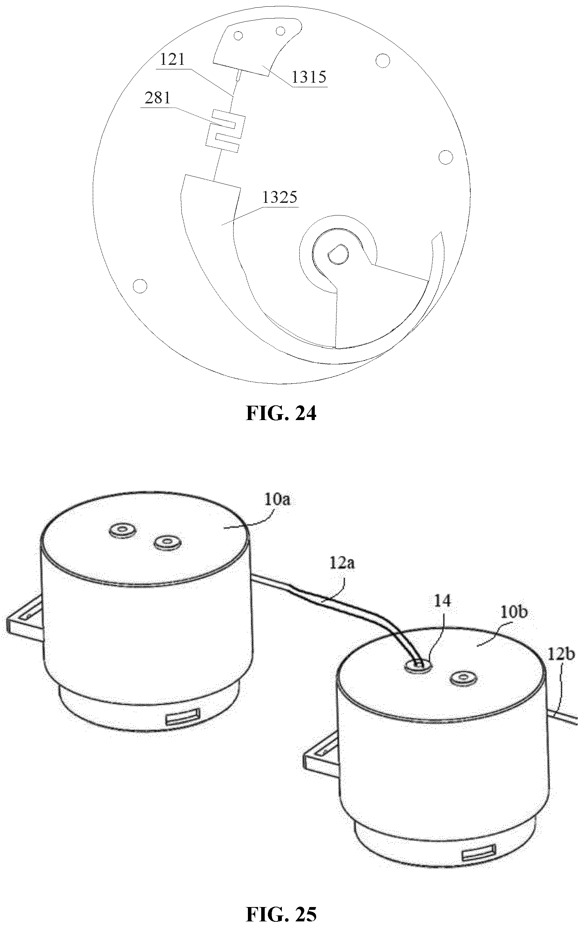

Additional sensors may be provided to further improve the driving control accuracy. For example, but not limited to, a force sensor 281 is provided on the drive wire 121 between the first constraining member 1315 and the second constraining member 1325, see FIG. 24. Moreover, a position sensor 282 is provided on the second drive component 22, see FIG. 21. During the operation, the pulling force and the rotation amount output by the drive can be fed back to a controller, and precise adjustment and control can be performed accordingly.

In addition to the above assembly relationship between the flexible instrument and the instrument drive device, in other application scenarios, the flexible instrument and the instrument drive device according to the present embodiment may be used in combination. Please refer to FIG. 25, which is a schematic view of the flexible instruments according to an embodiment of the present application being used in combination.

Two flexible instruments are shown in the drawing, where a first flexible instrument 10a is configured to be assembled with the instrument drive device to establish a power transmission relationship, and a second flexible instrument 10b is configured to provide the actuator for treating lesions. A first actuator unit 12a of the first flexible instrument 10a is connected to a docking unit 14 of the second flexible instrument 10b, and is connected to a second actuator unit 12b of the second flexible instrument 10b through an internal passage of the second flexible instrument 10b. The two flexible instruments cooperate with each other to realize coaxial conveying the flexible instruments.

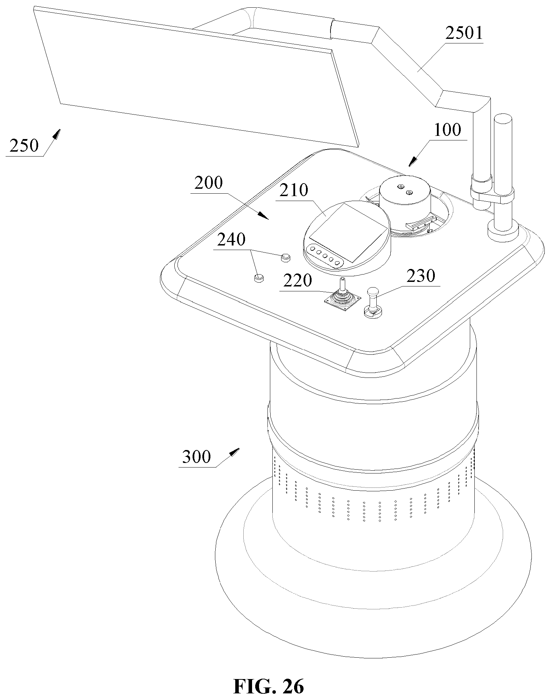

Apart from the above flexible surgical instrument, a surgical robot system is further provided according to the present embodiment. Please refer to FIG. 26, which is a schematic view showing the overall structure of a bedside therapist robot that can perform surgical treatment according to the embodiment of the present application.

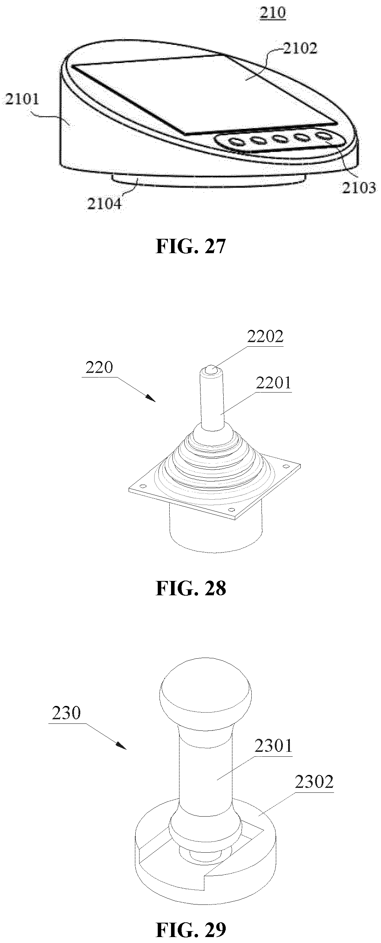

The surgical robot system includes a human-machine interaction system 200, a machine body 300 and an electrical system 400, and the machine body 300 is provided with the flexible surgical instrument 100 described above. The human-machine interaction system 200 includes a touch screen device 210, a mechanical handle 220, a wireless handle 230, a mechanical button 240 and a multi-degree-of-freedom display device 250. Please refer to FIGS. 27, 28 and 29. FIG. 27 is a schematic view showing the structure of the touch screen device according to the embodiment of the present application, FIG. 28 is a schematic view showing the structure of the mechanical handle according to the embodiment of the present application, and FIG. 29 is a schematic view showing the structure of the wireless handle according to the embodiment of the present application.

As shown in FIG. 27, the touch screen device 210 includes a support body 2101, a display component 2102, a touch component 2103 and a base 2104. The support body 2101 is arranged on the machine body 300 through the base 2104, and is rotatable relative to the base 2104. The display component 2102 and the touch component 2103 are arranged on an inclined top surface of the support body 2101, where an inclination angle can be specifically adjusted by an internal angle adjustment device, for example, the inclination angle may be adjusted in, but not limited to, a range of 10° to 60°. This angle adjustment may cooperate with the lifting of the entire machine body to adapt to the angle of the touch operation of the operator. The display component 2102 is used to display a current state of the robot and information about interaction with the tissue. The touch component 2103 is configured to input an operation command to adjust the state of the robot, and to control the operations such as caster locking, robot lifting, angle adjustment of the touch device, rotation of the touch device and rotation of an upper housing through touch buttons distributed horizontally.

As shown in FIG. 28, the mechanical handle 220 includes a handle joystick 2201 and a handle button 2202. The handle joystick 2201 is used to manipulate conveying of the instrument, i.e., to control the first drive component 21 of the flexible surgical instrument 100. The handle button 2202 is used to control functions of the instrument, i.e., to control the second drive component 22 and the third drive component 23 of the flexible surgical instrument 100.

As shown in FIG. 29, the wireless handle 230 includes a manipulation handle 2301 and a charging base 2302. The manipulation handle 2301 is wirelessly connected to the control system to realize the manipulation and control of the device. Herein, the wireless handle 230 may serve as a redundancy of the mechanical handle 220, and may be specifically provided according to the product design requirements. In the present embodiment, the charging base 2302 is a wireless charging base, and is arranged on an operation plane of the upper housing of the machine body by magnetic attraction. In this way, the charging position can be moved within a certain area to avoid interference with the arm of the operator in some operation postures of the robot.

Two mechanical buttons 240 are provided, which serve as functional redundant operation interfaces to control the two functions of the device respectively, that is, pulling or rotating the drive wire 121 of the actuator unit 12 to realize the operation functions such as rotation, or opening and closing of the actuator.

The display device 250 is connected to the machine body 300 through a multi-section arm support 2501, and the display angle of the display screen can be adjusted by adjusting any combination of distance and angle within the space determined by the support.

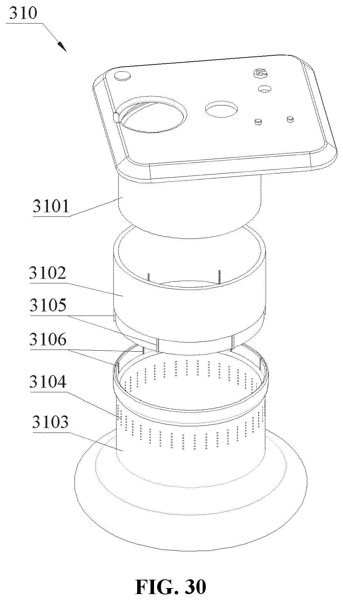

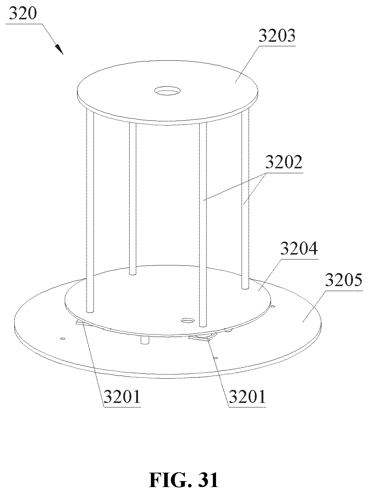

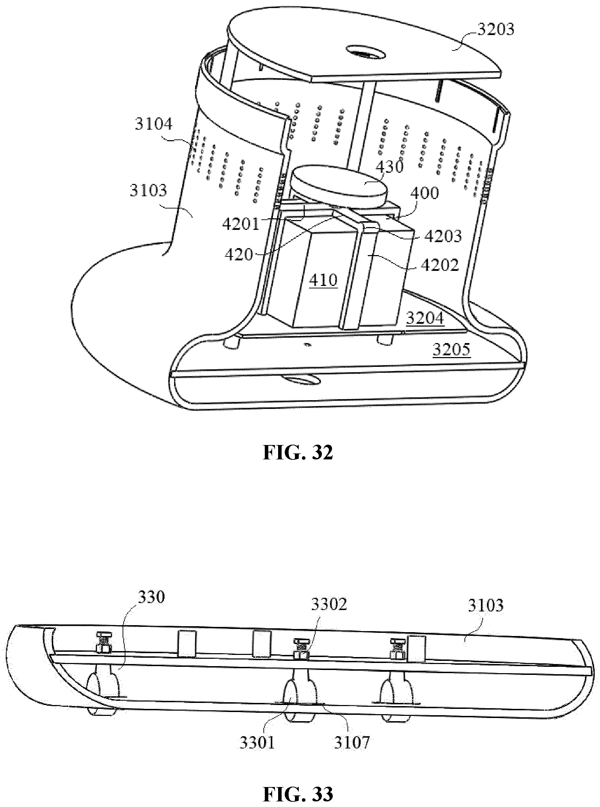

In the present embodiment, the machine body 300 includes a housing device 310, a lifting device 320 and a caster moving device 330. Please refer to FIGS. 26, 30, 31, 32 and 33. FIG. 30 is an exploded view showing the assembly of the machine body according to an embodiment of the present application. FIG. 31 is a schematic view showing the structure of the lifting device according to an embodiment of the present application. FIG. 32 is a schematic view showing the assembly relationship among the electrical system, the lifting device and a lower housing according to an embodiment of the present application. FIG. 33 is a schematic view showing the assembly relationship of the caster moving device according to an embodiment of the present application.

As a basic component, the housing device 310 is used for mounting of the lifting device 320 and the caster moving device 330, and the electrical system 400 is mounted inside the housing device 310, thereby forming an integrated skeleton system.

As shown in FIG. 30, the housing device 310 includes three parts, namely an upper housing 3101, an intermediate housing 3102 and a lower housing 3103. The upper housing 3101 is inserted in the intermediate housing 3102, and is in transition fit with the intermediate housing 3102. The upper housing 3101 is arranged on a lifting disc of the lifting device 320 to move up and down. Moreover, the upper housing can rotate around a central axis.

Herein, a top of the upper housing 3101 is an operation plane for placing the human-machine interaction system 200, and a bottom of the upper housing is provided with a wiring through hole to facilitate an electrical connection and a signal connection between the human-machine interaction system 200 and the electrical system 400. In the present embodiment, a lower portion of the intermediate housing 3102 is provided with a tooth portion 3105 extending along the axial direction, and an upper portion of the lower housing 3103 is provided with a groove portion 3106 extending along the axial direction. The intermediate housing and the lower housing mate with each other through the tooth and the groove, which facilitates assembly and maintenance. When the upper housing rotates around the central axis, the intermediate housing and the lower housing are fixed to each other due to the circumferential limiting pair formed by the tooth portion and groove portion mating with each other. In other words, the intermediate housing will not rotate relative to the lower housing. In other specific implementations, the circumferential limiting pair between the intermediate housing 3102 and the lower housing 3103 may be in other structural forms. Alternatively, the intermediate housing 3102 and the lower housing 3103 may form an integrated structure.

As shown in FIG. 31, the lifting device 320 includes a lifting drive component 3201, lifting leadscrews 3202, a lifting disc 3203, a motor fixing disc 3204 and a load-bearing base disc 3205. The lifting leadscrews 3202 are arranged between the lifting disc 3203 and the motor fixing disc 3204. A predetermined distance is provided between the motor fixing disc 3204 and the load-bearing base disc 3205 to form a mounting space for the lifting drive component 3201.

Accordingly, the lifting drive component 3201 is arranged below the motor fixing disc 3204 to drive a leadscrew in the lifting leadscrew 3202, so as to drive the lifting disc 3203 located at a top of the lifting leadscrew 3202 to move up and down, and drive the flexible surgical instrument 100 and the touch screen device 210 arranged on the upper housing 3101 via the upper housing 3101 to move correspondingly. Herein, each lifting leadscrew 3202 is provided with a lifting drive component 3201. In other specific applications, the leadscrews (not shown in the drawings) inside the lifting leadscrews 3202 can be synchronously driven by one lifting drive component. It should be understood that the power transmission relationship between the lifting drive component (a drive motor) and the leadscrew can be realized based on the conventional technologies, which will not be described in detail herein.

In the use state, the operator can output commands through the buttons of the touch component 2103 of the touch screen device 210 to control the lifting drive component 3201 to drive the leadscrews in the lifting leadscrews 3202 to rotate, to make the lifting disc 3203 supported by the lifting leadscrews 3202 be lifted and lowered. For example, but not limited to, a limiting mechanism (not shown in the drawings) may be provided to prevent the lifting distance of the lifting device from reaching a critical value, so as to ensure that the robot maintains a reliable use state during the surgery.

As shown in FIG. 32, the electrical system 400 according to the present embodiment is fixed in the lower housing 3103, and heat dissipation holes 3104 are provided in the lower housing 3103, which also functions as an electrical cabinet of an industrial computer. Specifically, a lower portion of the lower housing 3103 is of a round basin shape, and the load-bearing base disc 3205 of the lifting device 320 is inserted in a side wall thereof to be fixedly mounted. The caster moving device 330 may be fixed on the load-bearing base disc 3205.

As shown in FIG. 33, the caster moving device 330 includes casters 3301 and caster locks 3302. An opening 3107 is provided in a bottom plate of the lower housing 3103 at a position corresponding to each caster 3301. Part of a wheel body of the caster 3301 is arranged in the lower housing 3103. For example, but not limited to, most of the caster is covered by the bottom plate of the lower housing 3103. In this case, a size of the opening 3107 at the bottom of the lower housing may be slightly smaller than a wheel diameter of the caster 3301, resulting in a dustproof and soundproof effect. With this arrangement, on the one hand, the noises in the robot and caused by the caster movement can be blocked, and on the other hand, dust can be effectively prevented from entering the robot.

In the use state, the operator can output commands through the touch component 2103 of the touch screen device 210 to control the caster lock 3302 to lock the caster 3301. Moreover, the caster 3301 is provided with a self-locking electric feedback interface, which can output a caster state signal to a control device 410 of the electrical system 400. When the caster state signal indicates that the caster 3301 is in an unlocked state, the control device 410 prevents a starting command from being output to the instrument drive device 20. In this way, when the caster 3301 is not secured and self-locked, the instrument drive device cannot be started. That is, the instrument drive device can only be started when the caster 3301 is locked.

As shown in FIG. 32, the electrical system 400 includes the control device 410, a fixing device 420 and a cooling fan 430. The fixing device 420 is used to fix the control device 410, that is, the electrical control system device of the industrial computer. As shown in the drawings, the fixing device 420 includes multiple fixing claws 4201 and a rotatable claw 4202 located on a side of the fixing device. The rotatable claw 4202 is rotatably connected to the fixing claws 4201 through a damping rotation shaft 4203.

A lower end of each fixing claw 4201 is fixed on the load-bearing base disc 3205 in the machine body 300. Based on the rotation pair formed by the damping rotation shaft 322, the rotatable claw 4202 can rotate around the damping rotation shaft 322. When the rotatable claw is fixed, the electrical system 400 is completely fixed. When the rotatable claw 4202 rotates upward by an angle greater than or equal to 90°, the industrial computer gains one degree of freedom on the side where the rotatable claw is, and thus the electrical system 400 can be disassembled.

The cooling fan 430 is arranged above the control device 410, and with the assist of the heat dissipation holes 3104 provided in the lower housing 3103, the airflow formed by operation of the cooling fan 430 can cause convection of the hot air above the control device 410 and dissipate the heat to the outside through the surrounding heat dissipation holes 3104.

Based on the flexible surgical instrument 100 according to the present embodiment, it is possible to combine various instruments of the surgical robot system, and achieve the coaxial conveying function of the flexible instrument and the rotation, opening and closing operations of the actuator. In actual operation, with the human-machine interaction system of the surgical robot system, the functions are integrated, the operations are simplified, and the entire set of operations can be performed by one person alone without the cooperation of other medical personnel.

In actual use, by controlling the inclination of the touch panel and the coordination of the overall lifting, the robot can adapt to the using habits of different operators within an adjustable range of height, thereby making the operations comfortable and stable. Further, the adjustment parameters can be archived in the control system based on the user IDs to save the adjustment time for each time of operation of the operator.

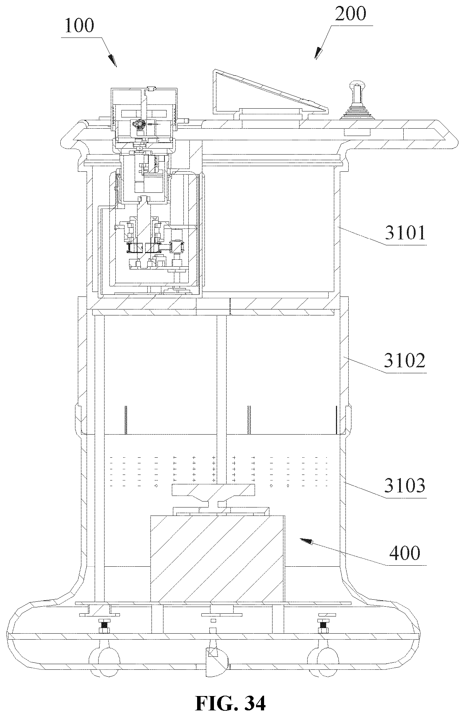

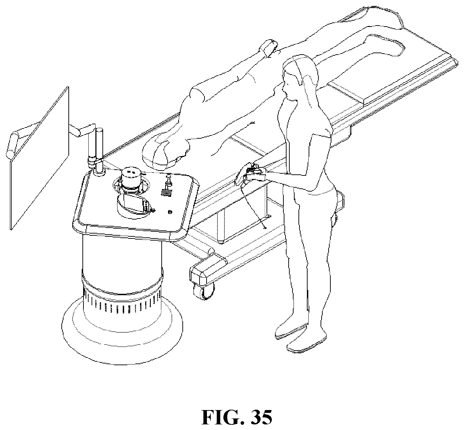

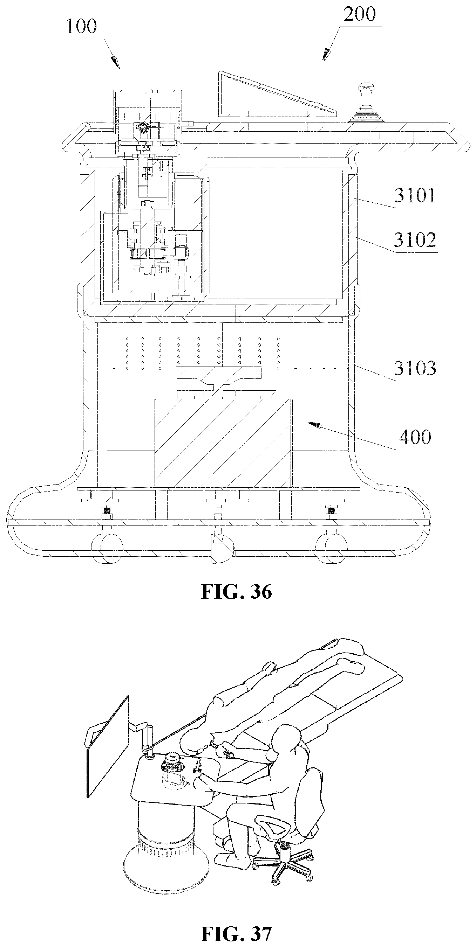

Please refer to FIGS. 34 and 35. FIG. 34 is a schematic view showing a state where the surgical robot system is lifted according to an embodiment of the present application, and FIG. 35 is a view showing a scenario where a doctor uses the robot while standing. Please refer to FIGS. 36 and 37. FIG. 36 is a schematic view showing a state where the surgical robot system is lowered according to an embodiment of the present application, and FIG. 37 is a view showing another scenario where a doctor uses the robot while seated. With the surgical robot system according to the present embodiment, the operations of the entire process can be performed by one person alone without the cooperation of other medical personnel, thereby solving the practical problems of high difficulty of single-person operation and high communication costs for coordinated operation in the current diagnosis and treatment during the natural orifice transluminal endoscopic surgery.

It should be understood that, other functional components of the surgical robot system, such as the electrical system and cooling fan, may be implemented based on the conventional technologies, and thus will not be described in detail herein.

The ordinal numbers "first" and "second" used herein are only intended to describe the components or structures of the same function in the technical solutions. It is understandable that, the use of the ordinal numbers "first" and "second" does not constitute a limitation on the understanding of the technical solutions claimed for protection in this application.

The embodiments described hereinabove are only preferred embodiments of the present application. It should be noted that, for those skilled in the art, several modifications and improvements may be made without departing from the principle of the present application.

## Claims

1. A flexible surgical instrument (100), comprising:
a flexible instrument (10); and
an instrument drive device (20) that is configured to output a driving force to the flexible instrument (10), wherein
the flexible instrument (10) comprises an instrument conveying unit (11), an actuator unit (12) and a transmission unit (13), wherein
the instrument conveying unit (11) comprises a housing (111) and an instrument storage component (112), the housing (111) has an internal accommodating space, an instrument outlet (1111) is provided on a side wall of the housing (111), an outer peripheral surface of the instrument storage component (112) is provided with a spiral accommodating groove (1121) configured for accommodating a flexible body of the actuator unit (12) in a wound manner, and at least part of the instrument storage component (112) is arranged inside the housing (111) and is rotatable and axially movable relative to the housing (111);
the actuator unit (12) comprises an actuator (122) and the flexible body, the flexible body comprises a drive wire (121) and a sleeve tube (123) in which the drive wire (121) is inserted, and the actuator (122) is arranged at a distal end of the drive wire (121);
the transmission unit (13) comprises a transmission base plate (133) that is drivingly connected to the instrument drive device (20), and the transmission base plate (133) is connected to the instrument storage component (112) of the instrument conveying unit (11) to drive the instrument storage component (112) to rotate and move axially relative to the housing (111); and
the transmission unit (13) further comprises an actuation transmission assembly (131) and a rotation transmission assembly (132) that are arranged on the transmission base plate (133), wherein the actuation transmission assembly (131) is configured to be driven by the instrument drive device (20) to extend or retract the drive wire (121), and the rotation transmission assembly (132) is configured to be driven by the instrument drive device (20) to rotate the drive wire (121),
wherein the instrument drive device (20) comprises a first drive component (21), an output shaft (265) and a drive base plate (241);
the drive base plate (241) mates with the transmission base plate (133) of the transmission unit (13), the first drive component (21) comprises an output end for outputting a rotational driving force, the output shaft (265) is drivingly connected to the output end of the first drive component (21), and the drive base plate (241) is connected to the output shaft (265); and
two power transmission paths are provided between the output end of the first drive component (21) and the output shaft (265), and are configured for driving the output shaft (265) to rotate and move axially, respectively, to drive the flexible instrument (10) to perform a conveying operation through the drive base plate (241).

2. The flexible surgical instrument (100) according to claim 1, further comprising:
a docking unit (14) for connecting with an external device, wherein
the docking unit (14) comprises an electrical connection port (141), a water connection port (142) and a guide tube (15), the electrical connection port (141) and the water connection port (142) are arranged at a top of the housing (111) of the instrument conveying unit (11), and the guide tube (15) is fixed in the housing (111), to allow a cable and a water pipe to be introduced to the actuator unit (12) through the electrical connection port (141), the water connection port (142) and the guide tube (15).

3. The flexible surgical instrument (100) according to claim 1, wherein
the instrument drive device (20) further comprises a second drive component (22), a third drive component (23), a first driving transmission disc (242) and a second driving transmission disc (243);
the first driving transmission disc (242) is drivingly connected to an output end of the second drive component (22), and the second driving transmission disc (243) is drivingly connected to an output end of the second drive component (22); and
the drive base plate (241) is provided with a first through hole (2411) and a second through hole (2412), the first through hole (2411) is arranged corresponding to the first driving transmission disc (242), and the second through hole (2412) is arranged corresponding to the second driving transmission disc (243), to allow the driving transmission discs drive the actuation transmission assembly (131) and the rotation transmission assembly (132) through the corresponding through holes, respectively.

4. The flexible surgical instrument (100) according to claim 3, wherein
the first driving transmission disc (242) and the second driving transmission disc (243) are fixed on two sliding supports (253), respectively, and each sliding support (253) is axially movable relative to a fixed slide rail (254) provided corresponding to the sliding support (253); and
an elastic reset element (255) is arranged between each sliding support (253) and a fixed structure, and the elastic reset element (255) is configured to be deformed when the sliding support (253) moves toward the instrument drive device (20), to apply a reset force to the sliding support (253).

5. The flexible surgical instrument (100) according to claim 1, wherein an engagement member (244) is provided on the drive base plate (241), an engagement groove (1311) is provided on the transmission base plate (133), and the engagement member (244) is placed in the engagement groove (1311) to form a circumferential rotation limiting pair to transmit the rotational driving force to the instrument through the engagement member (244) and the engagement groove (1311) mating with each other.

6. The flexible surgical instrument (100) according to claim 5, wherein
the engagement member (244) comprises a hook head portion (2441) extending outward from a body of the engagement member (244), a side wall of the instrument storage component (112) is provided with an engagement opening (1123) mating with the hook head portion (2441), and the engagement member (244) is configured to slide relative to the drive base plate (241) to switch between an extended work position and a retracted work position; and wherein
in a case that the engagement member (244) is at the extended work position, the hook head portion (2441) of the engagement member (244) is inserted in the engagement opening (1123); and
in a case that the engagement member (244) is at the retracted working position, the hook head portion (2441) of the engagement member (244) is disengaged from the engagement opening (1123).

7. A surgical robot system, comprising:
a machine body (300); and
the flexible surgical instrument (100) according to any one of claims 1 to 6, wherein the flexible surgical instrument (100) is arranged on the machine body (300).

8. The surgical robot system according to claim 7, further comprising:
a human-machine interaction system and an electrical system (400), wherein
the human-machine interaction system comprises a touch screen device (210), a mechanical handle (220) and a display device (250), the touch screen device (210) and the mechanical handle (220) are arranged at a top of the machine body (300), an inclination angle of a display component (2102) of the touch screen device (210) is adjustable, and the display device (250) is connected to the machine body (300) through a multi-section arm support.

9. The surgical robot system according to claim 8, wherein the touch screen device (210) further comprises a wireless handle (230) and a mechanical button (240) that are arranged at the top of the machine body (300), and the wireless handle (230) comprises a manipulation handle (2301) and a charging base (2302).

10. The surgical robot system according to claim 9, wherein
the machine body (300) comprises a housing device (310), a lifting device (320) and a caster moving device (330), wherein
the housing device (310) comprises an upper housing (3101) located at a top of the housing device (310) and a lower housing (3103) located at a bottom of the housing device (310), the lifting device (320) is arranged in the lower housing (3103), the lifting device (320) comprises a lifting disc (3203) that is movable upward and downward, and the upper housing (3101) is arranged on the lifting disc (3203) of the lifting device (320).

11. The surgical robot system according to claim 10, wherein
the lifting device (320) comprises a lifting drive component (3201), a lifting leadscrew (3202), a motor fixing disc (3204) and a load-bearing base disc (3205), wherein
the lifting leadscrew (3202) is arranged between the lifting disc (3203) and the motor fixing disc (3204), the load-bearing base disc (3205) is fixed below the motor fixing disc (3204), and a motor is arranged below the load-bearing base disc (3205) and is drivingly connected to the lifting leadscrew (3202).

12. The surgical robot system according to claim 10, wherein
the housing device (310) further comprises an intermediate housing (3102) located between the upper housing (3101) and the lower housing (3103), wherein
the upper housing (3101) is inserted in the intermediate housing (3102), and is in a transition fit with the intermediate housing (3102), and the upper housing (3101) is rotatable around a central axis relative to the intermediate housing (3102); and
a circumferential limiting pair is provided between a lower portion of the intermediate housing (3102) and an upper portion of the lower housing (3103), and the lower housing (3103) is provided with a heat dissipation hole (3104).

13. The surgical robot system according to claim 12, wherein the lower portion of the intermediate housing (3102) is provided with a tooth portion (3105) that extends axially, the upper portion of the lower housing (3103) is provided with a groove portion (3106) that extends axially, and the tooth portion (3105) and the groove portion (3106) mate with each other to form the circumferential limiting pair.

14. The surgical robot system according to any one of claims 10 to 13, wherein the caster moving device (330) comprises a caster (3301) and a caster lock (3302), the caster lock (3302) is arranged on the caster (3301), a bottom plate of the lower housing (3103) is provided with an opening corresponding to the caster (3301), and part of a wheel body of the caster (3301) is located inside the lower housing (3103).

15. The surgical robot system according to claim 14, wherein the caster (3301) is provided with a self-locking electrical feedback interface configured for outputting a caster state signal to a control device (410) of the electrical system (400), and the control device (410) is configured to prevent a starting command from being output to the instrument drive device (20) in a case that the caster state signal indicates that the caster (3301) is in an unlocked state.

16. The surgical robot system according to any one of claims 10 to 13, wherein a control device (410) of the electrical system (400) is arranged on a bottom plate of the lower housing (3103) through a fixing device (420), the fixing device (420) comprises a plurality of fixing claws (4201) and a rotatable claw (4202) that is located on a side of the fixing device (420), and the rotatable claw (4202) is rotatably connected to the fixing claws (4201) through a rotation shaft.

## Patentansprüche

1. Ein flexibles chirurgisches Instrument (100), umfassend:
ein flexibles Instrument (10); und
eine Instrumentenantriebsvorrichtung (20), die so ausgelegt ist, dass sie eine Antriebskraft an das flexible Instrument (10) abgibt, wobei
das flexible Instrument (10) eine Instrumentenfördereinheit (11), eine Aktuatoreinheit (12) und eine Übertragungseinheit (13) umfasst, wobei
die Instrumentenfördereinheit (11) ein Gehäuse (111) und eine Instrumentenaufbewahrungskomponente (112) umfasst, das Gehäuse (111) einen inneren Aufnahmeraum aufweist, an einer Seitenwand des Gehäuses (111) ein Instrumentenauslass (1111) vorgesehen ist, eine Außenumfangsfläche der Instrumentenaufbewahrungskomponente (112) mit einer spiralförmigen Aufnahmerille (1121) versehen ist, die dazu ausgelegt ist, einen flexiblen Körper der Aktuatoreinheit (12) in aufgewickelter Form aufzunehmen, und zumindest ein Teil der Instrumentenaufbewahrungskomponente (112) innerhalb des Gehäuses (111) angeordnet und relativ zum Gehäuse (111) drehbar sowie axial verschiebbar ist;
die Aktuatoreinheit (12) einen Aktuator (122) und den flexiblen Körper umfasst, wobei der flexible Körper einen Antriebsdraht (121) und ein Hülsenrohr (123) umfasst, in das der Antriebsdraht (121) eingeführt ist, und der Aktuator (122) an einem distalen Ende des Antriebsdrahts (121) angeordnet ist;
die Übertragungseinheit (13) eine Übertragungsgrundplatte (133) umfasst, die antriebsmäßig mit der Instrumentenantriebsvorrichtung (20) verbunden ist, und die Übertragungsgrundplatte (133) mit der Instrumentenaufbewahrungskomponente (112) der Instrumentenfördereinheit (11) verbunden ist, um die Instrumentenaufbewahrungskomponente (112) so anzutreiben, dass sie sich relativ zum Gehäuse (111) dreht und axial verschiebt; und
die Übertragungseinheit (13) ferner eine Betätigungsübertragungsbaugruppe (131) und eine Drehübertragungsbaugruppe (132) umfasst, die auf der Übertragungsgrundplatte (133) angeordnet sind, wobei die Betätigungsübertragungsbaugruppe (131) so ausgelegt ist, dass sie von der Instrumentenantriebsvorrichtung (20) angetrieben wird, um den Antriebsdraht (121) auszufahren oder einzufahren, und die Drehübertragungsbaugruppe (132) so ausgelegt ist, dass sie von der Instrumentenantriebsvorrichtung (20) angetrieben wird, um den Antriebsdraht (121) zu drehen, wobei die Instrumentenantriebsvorrichtung (20) eine erste Antriebskomponente (21), eine Abtriebswelle (265) und eine Antriebsgrundplatte (241) umfasst;
die Antriebsgrundplatte (241) mit der Übertragungsgrundplatte (133) der Übertragungseinheit (13) zusammenpasst, die erste Antriebskomponente (21) ein Abtriebsende zum Abgeben einer Drehantriebskraft umfasst, die Abtriebswelle (265) antriebsmäßig mit dem Abtriebsende der ersten Antriebskomponente (21) verbunden ist und die Antriebsgrundplatte (241) mit der Abtriebswelle (265) verbunden ist; und
zwischen dem Abtriebsende der ersten Antriebskomponente (21) und der Abtriebswelle (265) zwei Kraftübertragungswege vorgesehen sind, die so ausgelegt sind, dass sie die Abtriebswelle (265) jeweils drehen bzw. axial verschieben, um das flexible Instrument (10) über die Antriebsgrundplatte (241) zum Ausführen eines Fördervorgangs anzutreiben.

2. Das flexible chirurgische Instrument (100) nach Anspruch 1, ferner umfassend:
eine Andockeinheit (14) zum Verbinden mit einem externen Gerät, wobei
die Andockeinheit (14) einen elektrischen Anschluss (141), einen Wasseranschluss (142) und ein Führungsrohr (15) umfasst, der elektrische Anschluss (141) und der Wasseranschluss (142) an einer Oberseite des Gehäuses (111) der Instrumentenfördereinheit (11) angeordnet sind und das Führungsrohr (15) im Gehäuse (111) befestigt ist, um das Einführen eines Kabels und einer Wasserleitung zu der Aktuatoreinheit (12) durch den elektrischen Anschluss (141), den Wasseranschluss (142) und das Führungsrohr (15) zu ermöglichen.

3. Das flexible chirurgische Instrument (100) nach Anspruch 1, wobei
die Instrumentenantriebsvorrichtung (20) ferner eine zweite Antriebskomponente (22), eine dritte Antriebskomponente (23), eine erste Antriebsübertragungsscheibe (242) und eine zweite Antriebsübertragungsscheibe (243) umfasst;
die erste Antriebsübertragungsscheibe (242) antriebsmäßig mit einem Abtriebsende der zweiten Antriebskomponente (22) verbunden ist und die zweite Antriebsübertragungsscheibe (243) antriebsmäßig mit einem Abtriebsende der zweiten Antriebskomponente (22) verbunden ist; und
die Antriebsgrundplatte (241) mit einer ersten Durchgangsbohrung (2411) und einer zweiten Durchgangsbohrung (2412) versehen ist, wobei die erste Durchgangsbohrung (2411) entsprechend der ersten Antriebsübertragungsscheibe (242) angeordnet ist und die zweite Durchgangsbohrung (2412) entsprechend der zweiten Antriebsübertragungsscheibe (243) angeordnet ist, damit die Antriebsübertragungsscheiben die Betätigungsübertragungsbaugruppe (131) bzw. die Drehübertragungsbaugruppe (132) durch die entsprechenden Durchgangsbohrungen antreiben können.

4. Das flexible chirurgische Instrument (100) nach Anspruch 3, wobei
die erste Antriebsübertragungsscheibe (242) und die zweite Antriebsübertragungsscheibe (243) jeweils an zwei Gleitstützen (253) befestigt sind und jede Gleitstütze (253) relativ zu einer festen Gleitschiene (254), die entsprechend der Gleitstütze (253) vorgesehen ist, axial beweglich ist; und
ein elastisches Rückstellelement (255) zwischen jeder Gleitstütze (253) und einer feststehenden Struktur angeordnet ist, wobei das elastische Rückstellelement (255) so ausgebildet ist, dass es sich verformt, wenn sich die Gleitstütze (253) in Richtung der Instrumentenantriebsvorrichtung (20) bewegt, um eine Rückstellkraft auf die Gleitstütze (253) auszuüben.

5. Das flexible chirurgische Instrument (100) nach Anspruch 1, wobei an der Antriebsgrundplatte (241) ein Eingriffselement (244) vorgesehen ist, an der Übertragungsgrundplatte (133) eine Eingriffsnut (1311) vorgesehen ist und das Eingriffselement (244) in der Eingriffsnut (1311) angeordnet ist, um ein Umfangsdrehbegrenzungspaar zu bilden und die Drehantriebskraft über das Eingriffselement (244) und die miteinander zusammenpassende Eingriffsnut (1311) auf das Instrument zu übertragen.

6. Das flexible chirurgische Instrument (100) nach Anspruch 5, wobei
das Eingriffselement (244) einen Hakenkopfabschnitt (2441) umfasst, der sich von einem Körper des Eingriffselements (244) nach außen erstreckt, eine Seitenwand der Instrumentenaufbewahrungskomponente (112) mit einer Eingriffsöffnung (1123) versehen ist, die mit dem Hakenkopfabschnitt (2441) zusammenpasst, und das Eingriffselement (244) so ausgebildet ist, dass es relativ zur Antriebsgrundplatte (241) gleiten kann, um zwischen einer ausgefahrenen Arbeitsposition und einer zurückgezogenen Arbeitsposition umzuschalten; und wobei
sich der Hakenkopfabschnitt (2441) des Eingriffselements (244), wenn sich das Eingriffselement (244) in der ausgefahrenen Arbeitsposition befindet, in der Eingriffsöffnung (1123) befindet; und
der Hakenkopfabschnitt (2441) des Eingriffselements (244), wenn sich das Eingriffselement (244) in der zurückgezogenen Arbeitsposition befindet, aus der Eingriffsöffnung (1123) gelöst ist.

7. Ein chirurgisches Robotersystem, umfassend:
einen Maschinenkörper (300); und
das flexible chirurgische Instrument (100) gemäß einem der Ansprüche 1 bis 6, wobei das flexible chirurgische Instrument (100) am Maschinenkörper (300) angeordnet ist.

8. Das chirurgische Robotersystem nach Anspruch 7, ferner umfassend:
ein Mensch-Maschine-Interaktionssystem und ein elektrisches System (400), wobei
das Mensch-Maschine-Interaktionssystem eine Touchscreen-Vorrichtung (210), einen mechanischen Handgriff (220) und eine Anzeigevorrichtung (250) umfasst, wobei die Touchscreen-Vorrichtung (210) und der mechanische Handgriff (220) an der Oberseite des Maschinenkörpers (300) angeordnet sind, ein Neigungswinkel einer Anzeigekomponente (2102) der Touchscreen-Vorrichtung (210) einstellbar ist und die Anzeigevorrichtung (250) über eine mehrteilige Armhalterung mit dem Maschinenkörper (300) verbunden ist.

9. Das chirurgische Robotersystem nach Anspruch 8, wobei die Touchscreen-Vorrichtung (210) ferner einen drahtlosen Griff (230) und eine mechanische Taste (240) umfasst, die an der Oberseite des Maschinenkörpers (300) angeordnet sind, und der drahtlose Griff (230) einen Betätigungsgriff (2301) und eine Ladestation (2302) umfasst.

10. Das chirurgische Robotersystem nach Anspruch 9, wobei
der Maschinenkörper (300) eine Gehäusevorrichtung (310), eine Hebevorrichtung (320) und eine Lenkrollenbewegungsvorrichtung (330) umfasst, wobei
die Gehäusevorrichtung (310) ein oberes Gehäuse (3101), das sich an der Oberseite der Gehäusevorrichtung (310) befindet, und ein unteres Gehäuse (3103), das sich an der Unterseite der Gehäusevorrichtung (310) befindet, umfasst, wobei die Hebevorrichtung (320) im unteren Gehäuse (3103) angeordnet ist, die Hebevorrichtung (320) eine Hebescheibe (3203) umfasst, die nach oben und unten bewegbar ist, und das obere Gehäuse (3101) auf der Hebescheibe (3203) der Hebevorrichtung (320) angeordnet ist.

11. Das chirurgische Robotersystem nach Anspruch 10, wobei
die Hebevorrichtung (320) eine Hebeantriebskomponente (3201), eine Hebespindel (3202), eine Motorbefestigungsscheibe (3204) und eine tragende Basisscheibe (3205) umfasst, wobei
die Hebespindel (3202) zwischen der Hebescheibe (3203) und der Motorbefestigungsscheibe (3204) angeordnet ist, die tragende Basisscheibe (3205) unterhalb der Motorbefestigungsscheibe (3204) befestigt ist und ein Motor unterhalb der tragenden Basisscheibe (3205) angeordnet und antriebsmäßig mit der Hebespindel (3202) verbunden ist.

12. Das chirurgische Robotersystem nach Anspruch 10, wobei
die Gehäusevorrichtung (310) ferner ein Zwischengehäuse (3102) umfasst, das sich zwischen dem oberen Gehäuse (3101) und dem unteren Gehäuse (3103) befindet, wobei
das obere Gehäuse (3101) in das Zwischengehäuse (3102) eingesetzt ist und mit dem Zwischengehäuse (3102) in einer Übergangspassung steht und das obere Gehäuse (3101) relativ zum Zwischengehäuse (3102) um eine Mittelachse drehbar ist; und
zwischen einem unteren Abschnitt des Zwischengehäuses (3102) und einem oberen Abschnitt des unteren Gehäuses (3103) ein Umfangsbegrenzungspaar vorgesehen ist und das untere Gehäuse (3103) mit einer Wärmeableitungsöffnung (3104) versehen ist.

13. Das chirurgische Robotersystem nach Anspruch 12, wobei der untere Abschnitt des Zwischengehäuses (3102) mit einem sich axial erstreckenden Zahnabschnitt (3105) versehen ist, der obere Abschnitt des unteren Gehäuses (3103) mit einem sich axial erstreckenden Nutabschnitt (3106) versehen ist und der Zahnabschnitt (3105) und der Nutabschnitt (3106) miteinander zusammenpassen, um das Umfangsbegrenzungspaar zu bilden.

14. Das chirurgische Robotersystem nach einem der Ansprüche 10 bis 13, wobei die Lenkrollenbewegungsvorrichtung (330) eine Lenkrolle (3301) und eine Lenkrollenarretierung (3302) umfasst, die Lenkrollenarretierung (3302) an der Lenkrolle (3301) angeordnet ist, eine Bodenplatte des unteren Gehäuses (3103) mit einer der Lenkrolle (3301) entsprechenden Öffnung versehen ist und sich ein Teil des Radkörpers der Lenkrolle (3301) innerhalb des unteren Gehäuses (3103) befindet.

15. Das chirurgische Robotersystem nach Anspruch 14, wobei die Lenkrolle (3301) mit einer selbstsichernden elektrischen Rückkopplungsschnittstelle versehen ist, die dazu ausgelegt ist, ein Lenkrollenzustandssignal an eine Steuervorrichtung (410) des elektrischen Systems (400) auszugeben, und die Steuervorrichtung (410) so ausgelegt ist, dass sie verhindert, dass ein Startbefehl an die Instrumentenantriebsvorrichtung (20) ausgegeben wird, falls das Lenkrollenzustandssignal anzeigt, dass sich die Lenkrolle (3301) in einem entriegelten Zustand befindet.

16. Das chirurgische Robotersystem nach einem der Ansprüche 10 bis 13, wobei eine Steuervorrichtung (410) des elektrischen Systems (400) über eine Befestigungsvorrichtung (420) an einer Bodenplatte des unteren Gehäuses (3103) angeordnet ist, die Befestigungsvorrichtung (420) mehrere Befestigungsklauen (4201) und eine drehbare Klaue (4202) umfasst, die sich an einer Seite der Befestigungsvorrichtung (420) befindet, und die drehbare Klaue (4202) über eine Drehwelle drehbar mit den Befestigungsklauen (4201) verbunden ist.

## Revendications

1. Instrument chirurgical flexible (100), comprenant :
un instrument flexible (10) ; et
un dispositif d'entraînement d'instrument (20) configuré pour fournir une force d'entraînement à l'instrument flexible (10), dans lequel
l'instrument flexible (10) comprend une unité de transport d'instrument (11), une unité d'actionneur (12) et une unité de transmission (13) ;
l'unité de transport d'instrument (11) comprend un boîtier (111) et un composant de rangement d'instrument (112), le boîtier (111) comporte un espace de réception interne, une sortie d'instrument (1111) est pourvue sur une paroi latérale du boîtier (111), une surface périphérique externe du composant de rangement d'instrument (112) est pourvue d'une rainure de réception hélicoïdale (1121) configurée pour recevoir un corps flexible de l'unité d'actionneur (12) de manière enroulée, et au moins une partie du composant de rangement d'instrument (112) est agencée à l'intérieur du boîtier (111) et est rotative et mobile axialement par rapport au boîtier (111) ;
l'unité d'actionneur (12) comprend un actionneur (122) et le corps flexible, le corps flexible comprend un câble d'entraînement (121) et un tube-manchon (123) dans lequel est inséré le câble d'entraînement (121), et l'actionneur (122) est agencé à une extrémité distale du câble d'entraînement (121) ;
l'unité de transmission (13) comprend une plaque de base de transmission (133) qui est connectée en entraînement au dispositif d'entraînement d'instrument (20), et la plaque de base de transmission (133) est connectée au composant de rangement d'instrument (112) de l'unité de transport d'instrument (11) pour entraîner la rotation et le déplacement axial du composant de rangement d'instrument (112) par rapport au boîtier (111) ;
l'unité de transmission (13) comprend en outre un ensemble de transmission d'actionnement (131) et un ensemble de transmission de rotation (132) agencés sur la plaque de base de transmission (133), dans lequel l'ensemble de transmission d'actionnement (131) est configuré pour être entraîné par le dispositif d'entraînement d'instrument (20) afin de déployer ou de rétracter le câble d'entraînement (121), et l'ensemble de transmission de rotation (132) est configuré pour être entraîné par le dispositif d'entraînement d'instrument (20) afin de faire tourner le câble d'entraînement (121) ;
le dispositif d'entraînement d'instrument (20) comprend un premier composant d'entraînement (21), un axe de sortie (265) et une plaque de base d'entraînement (241) ;
la plaque de base d'entraînement (241) s'accouple avec la plaque de base de transmission (133) de l'unité de transmission (13), le premier composant d'entraînement (21) comprend une extrémité de sortie pour fournir une force d'entraînement en rotation, l'axe de sortie (265) est connecté en entraînement à l'extrémité de sortie du premier composant d'entraînement (21), et la plaque de base d'entraînement (241) est connectée à l'axe de sortie (265) ; et
deux voies de transmission de puissance sont pourvues entre l'extrémité de sortie du premier composant d'entraînement (21) et l'axe de sortie (265), et sont configurées pour entraîner respectivement l'axe de sortie (265) en rotation et en translation axiale, afin d'entraîner l'instrument flexible (10) pour effectuer une opération de transport à travers la plaque de base d'entraînement (241).

2. Instrument chirurgical flexible (100) selon la revendication 1, comprenant en outre :
une unité d'accueil (14) pour assurer une connexion avec un dispositif externe, dans lequel
l'unité d'accueil (14) comprend un port de connexion électrique (141), un port de connexion d'eau (142) et un tube de guidage (15), le port de connexion électrique (141) et le port de connexion d'eau (142) sont agencés dans la partie supérieure du boîtier (111) de l'unité de transport d'instrument (11), et le tube de guidage (15) est fixé dans le boîtier (111) pour permettre l'introduction d'un câble et d'un tuyau d'eau dans l'unité d'actionneur (12) à travers le port de connexion électrique (141), le port de connexion d'eau (142) et le tube de guidage (15).

3. Instrument chirurgical flexible (100) selon la revendication 1, dans lequel
le dispositif d'entraînement d'instrument (20) comprend en outre un deuxième composant d'entraînement (22), un troisième composant d'entraînement (23), un premier disque de transmission d'entraînement (242) et un deuxième disque de transmission d'entraînement (243) ;
le premier disque de transmission d'entraînement (242) est connecté en entraînement à une extrémité de sortie du deuxième composant d'entraînement (22), et le deuxième disque de transmission d'entraînement (243) est connecté en entraînement à une extrémité de sortie du deuxième composant d'entraînement (22) ; et
la plaque de base d'entraînement (241) est pourvue d'un premier trou traversant (2411) et d'un deuxième trou traversant (2412), le premier trou traversant (2411) est agencé en correspondance avec le premier disque de transmission d'entraînement (242), et le deuxième trou traversant (2412) est agencé en correspondance avec le deuxième disque de transmission d'entraînement (243) afin de permettre aux disques de transmission d'entraînement d'entraîner l'ensemble de transmission d'actionnement (131) et l'ensemble de transmission de rotation (132) à travers les trous traversants correspondants, respectivement.

4. Instrument chirurgical flexible (100) selon la revendication 3, dans lequel
le premier disque de transmission d'entraînement (242) et le deuxième disque de transmission d'entraînement (243) sont fixés respectivement sur deux supports coulissants (253), et chaque support coulissant (253) est mobile axialement par rapport à un rail de coulissement fixe (254) pourvu en correspondance avec le support coulissant (253) ; et
un élément de rappel élastique (255) est agencé entre chaque support coulissant (253) et une structure fixe, et l'élément de rappel élastique (255) est configuré pour se déformer quand le support coulissant (253) se déplace vers le dispositif d'entraînement d'instrument (20) afin d'appliquer une force de rappel au support coulissant (253) .

5. Instrument chirurgical flexible (100) selon la revendication 1, dans lequel un élément d'engagement (244) est pourvu sur la plaque de base d'entraînement (241), une rainure d'engagement (1311) est pourvue sur la plaque de base de transmission (133), et l'élément d'engagement (244) est placé dans la rainure d'engagement (1311) pour former une paire de limitation de rotation circonférentielle afin de transmettre la force d'entraînement en rotation à l'instrument via l'accouplement mutuel de l'élément d'engagement (244) et de la rainure d'engagement (1311).

6. Instrument chirurgical flexible (100) selon la revendication 5, dans lequel
l'élément d'engagement (244) comprend une portion de tête en forme de crochet (2441) se déployant vers l'extérieur à partir du corps de l'élément d'engagement (244), une paroi latérale du composant de rangement d'instrument (112) est pourvue d'une ouverture d'engagement (1123) qui s'accouple avec la portion de tête en forme de crochet (2441), et l'élément d'engagement (244) est configuré pour coulisser par rapport à la plaque de base d'entraînement (241) afin de commuter entre une position de travail déployée et une position de travail rétractée ; et dans lequel
dans le cas où l'élément d'engagement (244) se trouve dans la position de travail déployée, la portion de tête en forme de crochet (2441) de l'élément d'engagement (244) est insérée dans l'ouverture d'engagement (1123) ; et
dans le cas où l'élément d'engagement (244) se trouve dans la position de travail rétractée, la portion de tête en forme de crochet (2441) de l'élément d'engagement (244) est désengagée de l'ouverture d'engagement (1123).

7. Système de robot chirurgical, comprenant :
un corps de machine (300) ; et
l'instrument chirurgical flexible (100) selon l'une quelconque des revendications 1 à 6, dans lequel l'instrument chirurgical flexible (100) est agencé sur le corps de machine (300).

8. Système de robot chirurgical selon la revendication 7, comprenant en outre :
un système d'interaction homme-machine et un système électrique (400), dans lequel
le système d'interaction homme-machine comprend un dispositif à écran tactile (210), une poignée mécanique (220) et un dispositif d'affichage (250), le dispositif à écran tactile (210) et la poignée mécanique (220) sont agencés dans la partie supérieure du corps de machine (300), l'angle d'inclinaison d'un composant d'affichage (2102) du dispositif à écran tactile (210) est réglable, et le dispositif d'affichage (250) est connecté au corps de machine (300) par l'intermédiaire d'un support de bras à sections multiples.

9. Système de robot chirurgical selon la revendication 8, dans lequel le dispositif à écran tactile (210) comprend en outre une poignée sans fil (230) et un bouton mécanique (240) agencés dans la partie supérieure du corps de machine (300), et la poignée sans fil (230) comprend une poignée de manipulation (2301) et une base de chargement (2302).

10. Système de robot chirurgical selon la revendication 9, dans lequel
le corps de machine (300) comprend un dispositif de boîtier (310), un dispositif de levage (320) et un dispositif de déplacement à roulettes (330), et
le dispositif de boîtier (310) comprend un boîtier supérieur (3101) situé dans la partie supérieure du dispositif de boîtier (310) et un boîtier inférieur (3103) situé dans la partie inférieure du dispositif de boîtier (310), le dispositif de levage (320) est agencé dans le boîtier inférieur (3103), le dispositif de levage (320) comprend un disque de levage (3203) qui est mobile vers le haut et vers le bas, et le boîtier supérieur (3101) est agencé sur le disque de levage (3203) du dispositif de levage (320).

11. Système de robot chirurgical selon la revendication 10, dans lequel
le dispositif de levage (320) comprend un composant d'entraînement de levage (3201), une vis-mère de levage (3202), un disque de fixation de moteur (3204) et un disque de base porteur (3205), et
la vis-mère de levage (3202) est agencée entre le disque de levage (3203) et le disque de fixation de moteur (3204), le disque de base porteur (3205) est fixé sous le disque de fixation de moteur (3204), et un moteur est agencé sous le disque de base porteur (3205) et est connecté en entraînement à la vis-mère de levage (3202).

12. Système de robot chirurgical selon la revendication 10, dans lequel
le dispositif de boîtier (310) comprend en outre un boîtier intermédiaire (3102) situé entre le boîtier supérieur (3101) et le boîtier inférieur (3103) ;
le boîtier supérieur (3101) est inséré dans le boîtier intermédiaire (3102) et est en ajustement de transition avec le boîtier intermédiaire (3102), et le boîtier supérieur (3101) peut tourner autour d'un axe central par rapport au boîtier intermédiaire (3102) ; et
une paire de limitation circonférentielle est pourvue entre une portion inférieure du boîtier intermédiaire (3102) et une portion supérieure du boîtier inférieur (3103), et le boîtier inférieur (3103) est pourvu d'un trou de dissipation de chaleur (3104).

13. Système de robot chirurgical selon la revendication 12, dans lequel la portion inférieure du boîtier intermédiaire (3102) est pourvue d'une portion de dent (3105) qui s'étend axialement, la portion supérieure du boîtier inférieur (3103) est pourvue d'une portion de rainure (3106) qui s'étend axialement, et la portion de dent (3105) et la portion de rainure (3106) s'accouplent pour former la paire de limitation circonférentielle.

14. Système de robot chirurgical selon l'une quelconque des revendications 10 à 13, dans lequel le dispositif de déplacement à roulettes (330) comprend une roulette (3301) et un verrou de roulette (3302), le verrou de roulette (3302) est agencé sur la roulette (3301), une plaque inférieure du boîtier inférieur (3103) est pourvue d'une ouverture correspondant à la roulette (3301), et une partie du corps de roue de la roulette (3301) est située à l'intérieur du boîtier inférieur (3103).

15. Système de robot chirurgical selon la revendication 14, dans lequel la roulette (3301) est pourvue d'une interface de retour électrique d'auto-verrouillage configurée pour envoyer un signal d'état de roulette à un dispositif de contrôle (410) du système électrique (400), et le dispositif de contrôle (410) est configuré pour empêcher l'envoi d'une commande de démarrage au dispositif d'entraînement d'instrument (20) dans le cas où le signal d'état de roulette indique que la roulette (3301) est dans un état déverrouillé.

16. Système de robot chirurgical selon l'une quelconque des revendications 10 à 13, dans lequel un dispositif de contrôle (410) du système électrique (400) est agencé sur une plaque inférieure du boîtier inférieur (3103) par l'intermédiaire d'un dispositif de fixation (420), le dispositif de fixation (420) comprend une pluralité de griffes de fixation (4201) et une griffe rotative (4202) située sur un côté du dispositif de fixation (420), et la griffe rotative (4202) est connectée de manière rotative aux griffes de fixation (4201) par l'intermédiaire d'un axe de rotation.
